(19) Europäisches Patentamt
European Patent Office
Office européen des brevets

(11) **EP 2 330 554 A1**

(12) **EUROPEAN PATENT APPLICATION**
published in accordance with Art. 153(4) EPC

(43) Date of publication:
**08.06.2011 Bulletin 2011/23**

(51) Int Cl.:
***G06Q 50/00*** (2006.01)     ***A61B 5/00*** (2006.01)

(21) Application number: **09814424.9**

(22) Date of filing: **12.08.2009**

(86) International application number:
**PCT/JP2009/064475**

(87) International publication number:
**WO 2010/032579 (25.03.2010 Gazette 2010/12)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **19.09.2008 JP 2008240520**

(71) Applicant: **Hitachi, Ltd.
Chiyoda-ku
Tokyo 100-8280 (JP)**

(72) Inventors:
• **KURIYAMA, Hiroyuki
Chiyoda-ku, Tokyo 100-8220 (JP)**
• **SHINTANI, Takahiko
Chiyoda-ku, Tokyo 100-8220 (JP)**
• **MOTOBAYASHI, Masahiro
Chiyoda-ku, Tokyo 100-8220 (JP)**

(74) Representative: **Strehl Schübel-Hopf & Partner
Maximilianstraße 54
D-80538 München (DE)**

(54) **METHOD AND SYSTEM FOR GENERATING HISTORY OF BEHAVIOR**

(57) Disclosed are method and system for generating history of behavior that is capable of simplifying input of a behavior content of a human behavior pattern determined from data measured by a sensor. A computer obtains biological information measured by a sensor which is mounted to a person and accumulates the biological information, obtains motion frequencies from the accumulated biological information, obtains time-series change points of the motion frequencies, extracts a period between the change points as a scene which is a period of being in the state of an identical motion, compares the motion frequencies with a preset condition for each extracted scene and identifies the action contents in the scene, estimates the behavior content performed by the person in the scene on the basis of the appearance sequence of the action contents, and generates the history of the behaviors on the basis of the estimated behavior contents.

```
              START
    S1
         COLLECT SENSOR DATA
    S2
            SPLIT SCENES
    S3
    LIST ACTIVITY DETAIL CANDIDATES
    S4
         PRIORITIZE ACTIVITY
         DETAIL CANDIDATES
                                    S5
                   DISPLAY
                                    S6
                   ENTER ACTIVITY DETAILS
    S7
    ESTABLISH ACTIVITY RECORD
    S8
       STORE ACTIVITY RECORD
                END
```

***FIG. 5***

EP 2 330 554 A1

# Description

Technical Field

This invention relates to a sensor network system that includes a sensor node for measuring living organism information. In particular, this invention relates to a technology of obtaining an activity history of a monitored subject with the use of a sensor node worn by the monitored subject, and analyzing an activity pattern of the monitored subject from the activity history.

Background Art

In recent years, expectations are put on recording and accumulating people's activity details in large quantity and analyzing the huge data, to thereby acquire new insights and provide a service. Its application has already been established on the Internet in the form of, for example, a mechanism for utilizing search keywords and purchase histories to send advertisements unique to each individual and thus recommend products that are likely to interest that person.

The same mechanism is conceivable in real life as well. Examples of possible applications include: recording and analyzing day-to-day work details to improve the business efficiency of the entire organization; recording a person's daily life to evaluate the person's diet, exercise, and the regularity of his/her daily routine and provide a health care service for preventing lifestyle-related diseases; and analyzing life records and purchase histories of a large number of people to present advertisements to people who live their lives in a particular life pattern and thus recommend products that have been purchased by many of those people.

Meanwhile, studies are being done on network systems in which a small-sized electronic circuit having a wireless communication function is added to a sensor to enter various types of real life information to an information processing device in real time. The sensor network systems have a wide range of possible applications. For example, a medical application has been proposed in which a small-sized electronic circuit with a wireless circuit, a processor, a sensor, and a battery integrated therein is used to constantly monitor acceleration or living organism information such as pulse and to transmit monitoring results to a diagnostic machine or the like through wireless communication, and healthiness is determined based on the monitoring results.

There has also been known a technology of evaluating work done by a worker by extracting a feature vector from measurement data of a sensor that is worn around the worker's wrist or on the worker's back (e.g., JP 2006-209468 A).

Another known technology involves installing a mat switch and a human sensor, or other sensors, in the home of a watched person, and analyzing in time series the life pattern of the watched person from data obtained through these different types of sensors (e.g., JP 2005-346291 A).

Still another known technology involves obtaining measurement data through a sensor, such as a pedometer, a thermometer, or a pulse sensor, that is worn by a user to analyze the activity pattern of the person at a time granularity specified by the user or by others (e.g., JP 2005-062963 A).

Other disclosed technologies include one in which the activity pattern of a user of a transmission terminal device is figured out from environment information received by the transmission terminal device (e.g., JP 2004-287539 A), and one in which the activity pattern of a person is detected from a vibration sensor worn on the person's body.

A technology of analyzing the activity pattern of a person based on data that is collected from a vibration sensor or the like is also known (e.g., JP 2008-000283 A).

Disclosure of the Invention

While it is expected that many useful services may be provided by recording and analyzing users' daily activities, it is a considerable chore for users to accurately record everyday activity details along with the time of the activities. The labor of recording is saved significantly by employing, for example, a method in which activities are automatically obtained through a sensor worn on a user's body.

The above-mentioned prior art examples are capable of automatically discriminating among general actions such as walking, exercising, and resting with regard to the activities of a user wearing a sensor node, but have difficulty in automatically identifying a concrete activity such as the user writing e-mail to a friend on a personal computer during a resting period. The resultant problem is that the user therefore needs to enter every detail of activities he/she has done during a resting period, and is required to expend much labor to enter the details of each and every activity. The term "action" here means the very act of a person moving his/her body physically, and the term "activity" here indicates a series of actions which is done by a person with an intent or a purpose. For instance, the action of a person walking to his/her workplace is "walking" and the activity of the person is "commuting".

Another problem of the prior art example, where a point of change in measurement data of the sensor is extracted as a point of change in action, is that simply segmenting activities at points of change in action lowers the accuracy of activity identification, because an activity of a person often involves a combination of a plurality of actions. For instance, an activity of a sleeping person may involve temporarily waking up to go to a bathroom or the like. If actions are to be determined simply from measurement data of the sensor, an action pattern involving sleeping followed by walking, resting, and walking is determined before returning to sleeping. In this case, activity identification based solely on points of change in action has a problem in that activities are segmented unnecessarily finely when the series of actions of walking, resting, and walking, should be associated with an activity of going to a bathroom.

This invention has been made in view of the above-mentioned problems, and an object of this invention is therefore to facilitate the entering of activity details based on information of human actions that are determined from measurement data of a sensor.

According to this invention, there is provided an activity history generating method of generating an activity history with a sensor, which is worn by a person to measure living organism information, and a computer, which obtains the living organism information from the sensor to identify an action state of the person, including the steps of: obtaining the living organism information by the computer and accumulating the living organism information on the computer; obtaining, by the computer, an action count from the accumulated living organism information; extracting, by the computer, a plurality of points of change in time series in the action count; extracting, by the computer, a period between the points of change as a scene in which the same action state is maintained; comparing, by the computer, the action count of each extracted scene against conditions set in advance to identify action details of the scene; estimating, by the computer, details of an activity that is done by the person during the scene based on an appearance order of the action details; and generating an activity history based on the estimated activity details.

Accordingly, this invention makes it easy for a user to enter activity details of each scene by extracting a scene from action states of a person, identifying action details for each scene, estimating activity details from the appearance order of the action details, and presenting the activity details to the user. This invention thus saves labor required to create an activity history.

This enables anyone to accomplish the hitherto difficult task of collecting detailed and accurate activity histories over a long period of time and, through activity analysis based on this information, new insights are obtained in various fields including work assistance, health care, and marketing, and services that are better matched to users' needs can be provided.

Brief Description of the Drawings

FIG. 1 is a block diagram illustrating an example of the configuration of a life log system to which this invention is applied.
FIG. 2 is a diagram illustrating an example of a bracelet type sensor node, with Part (a) of FIG. 2 being a schematic diagram viewed from the front of a bracelet type sensor node and Part (b) of FIG. 2 being a sectional view viewed from a side of the bracelet type sensor node.
FIG. 3 is a block diagram of an electronic circuit mounted to a substrate of the bracelet type sensor node.
FIG. 4 is a block diagram illustrating function elements of the life log system.
FIG. 5 is a flow chart illustrating the overall flow of processing that is executed in the life log system.
FIG. 6 is a flow chart illustrating an example of processing that is executed in a scene splitting module of a server.
FIG. 7 is a graph of a relation between acceleration and time, which shows an example of how a zero cross count is determined.
FIG. 8 is an explanatory diagram illustrating a format of data compiled for each given time interval.
FIG. 9 is a graph in which action counts per unit time are sorted in time series.
FIG. 10 is a flow chart illustrating an example of processing of setting action details of a user for each scene.
FIG. 11 is an explanatory diagram illustrating an example of a table of determination values which set a relation between the action count and the action details.
FIG. 12 is a flow chart illustrating an example of processing of combining a plurality of walking scenes.
FIG. 13 is a flow chart illustrating an example of processing of combining a plurality of sleeping scenes.
FIG. 14 is a graph showing a relation between the action count, scenes prior to combining, scenes after combining, and time.
FIG. 15 is an explanatory diagram illustrating an example of scene data containing action details, which is generated by an activity detail analyzing module.
FIG. 16 is a flow chart illustrating an example of processing of generating and prioritizing candidates for activity details which is executed by the activity detail analyzing module.
FIG. 17 is an explanatory diagram illustrating an example of a scene determining rule table.
FIG. 18 is a screen image of an activity history input window which is displayed on a display unit of a client computer.
FIG. 19 is an explanatory diagram illustrating the data structure of activity details.
FIG. 20 is a screen image of a candidate box which contains the candidates for activity details.
FIG. 21 is an explanatory diagram illustrating how candidates are selected manually.
FIG. 22 is an explanatory diagram illustrating an example of an activity detail storing table for storing an activity history.
FIG. 23 is an explanatory diagram illustrating an example of an activity detail item management table for storing activity detail items.

FIG. 24 is a screen image of a comment input window in a first modification example.

FIG. 25 is an explanatory diagram illustrating an example of an activity detail storing table for storing an activity history in the first modification example.

FIG. 26 is a screen image of a comment input window in a second modification example.

FIG. 27 is an explanatory diagram illustrating an example of an activity detail storing table for storing an activity history in the second modification example.

FIG. 28 is a screen image of an input window in a third modification example.

FIG. 29 is an explanatory diagram illustrating an example of an activity detail storing table for storing an activity history in the third modification example.

FIG. 30 is a block diagram illustrating function elements of a life log system in a fourth modification example.

Best Mode for carrying out the Invention

An embodiment of this invention is described below with reference to the accompanying drawings.

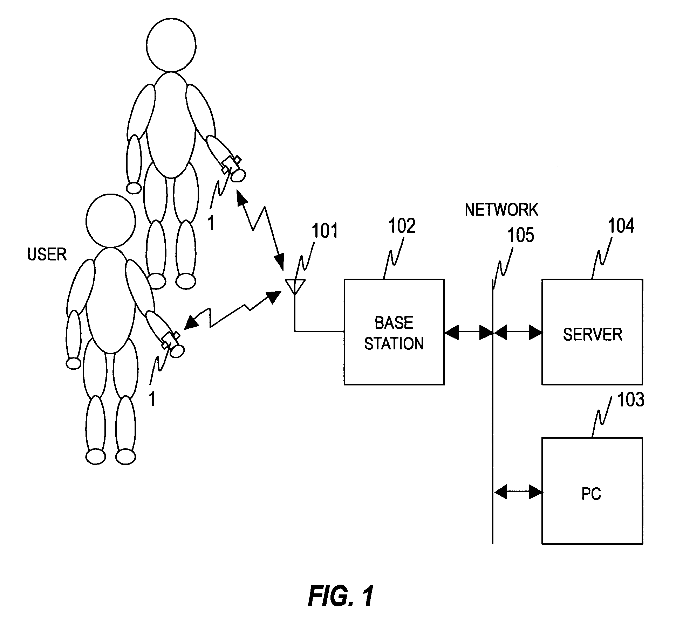

FIG. 1 is a block diagram illustrating an example of the configuration of a life log system to which this invention is applied. In the illustrated example, the life log system of this invention uses a bracelet type sensor node 1, which includes an acceleration sensor, as a sensor for detecting an action (or a state) of a user of the system, to detect the acceleration of an arm as living organism information. The bracelet type sensor node 1 is worn on an arm of the user (or a participant) to detect the arm's acceleration, and transmits the detected acceleration (hereinafter, referred to as sensing data) to a base station 102 in a given cycle.

In FIG. 1, the base station 102 communicates with a plurality of bracelet type sensor nodes 1 via an antenna 101 to receive from each bracelet type sensor node 1 sensing data that reflects the motion of the user, and transfers the sensing data to a server 104 over a network 105. The server 104 stores the received sensing data. The server 104 analyzes the sensing data received from the base station 102 and, as will be described later, generates and stores a life log which indicates an activity history of the user.

The life log generated by the server 104 can be viewed or edited on a client computer (PC) 103, which is operated by the user of the life log system. The user can add detailed information to the life log generated by the server 104.

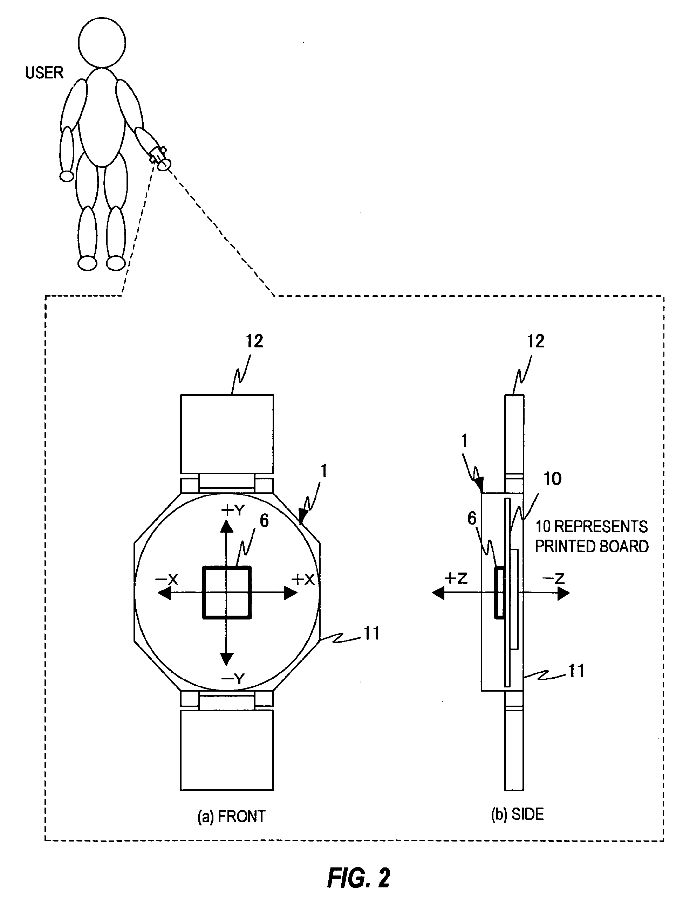

FIG. 2 is a diagram illustrating an example of the bracelet type (or wrist watch type) sensor node 1, which constitutes a sensor unit of the life log system of this invention. Part (a) of FIG. 2 is a schematic diagram viewed from the front of the bracelet type sensor node 1, and Part (b) of FIG. 2 is a sectional view viewed from a side of the bracelet type sensor node 1. The bracelet type sensor node 1 measures mainly the motion of the user (wearer).

The bracelet type sensor node 1 includes a case 11 which houses a sensor and a control unit, and a band 12 with which the case 11 is worn around a human arm.

As illustrated in Part (b) of FIG. 2, the case 11 houses therein a substrate 10, which includes a microcomputer 3, a sensor 6, and others. The illustrated example employs as the sensor 6 for measuring the motion of a human body (living organism) an acceleration sensor that measures the acceleration along three axes X-Y-Z in the drawing. The bracelet type sensor node 1 of this embodiment further includes a temperature sensor (not shown), which is used to measure the body temperature of the user, and outputs the measured body temperature along with the acceleration as sensing data.

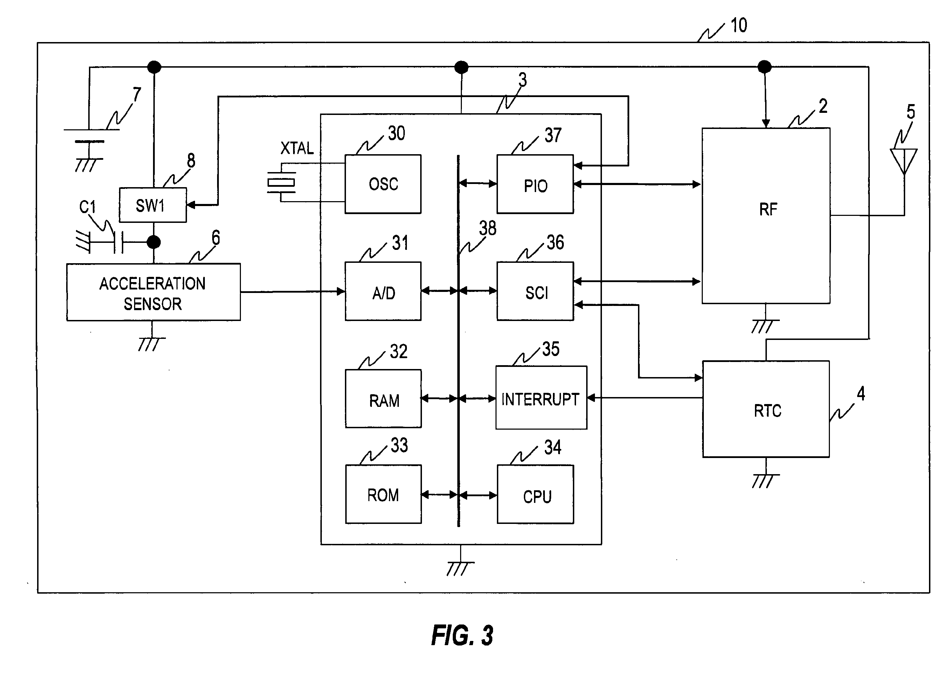

FIG. 3 is a block diagram of an electronic circuit mounted to the substrate 10 of the bracelet type sensor node 1. In FIG. 3, disposed on the substrate 10 are a wireless communication unit (RF) 2 which includes an antenna 5 to communicate with the base station 102, the microcomputer 3 which controls the sensor 6 and the wireless communication unit 2, a real time clock (RTC) 4 which functions as a timer for starting up the microcomputer 3 intermittently, a battery 7 which supplies electric power to the respective units, and a switch 8 which controls power supply to the sensor 6. A bypass capacitor C1 is connected between the switch 8 and the sensor 6 in order to remove noise and to avoid wasteful power consumption by lowering the speed of charging and discharging. Wasteful power consumption can be cut down by controlling the switch 8 in a manner that reduces the number of times of charging/discharging of the bypass capacitor C1.

The microcomputer 3 includes a CPU 34 which carries out arithmetic processing, a ROM 33 which stores programs and the like executed by the CPU 33, a RAM 32 which stores data and the like, an interrupt control unit 35 which interrupts the CPU 34 based on a signal (timer interrupt) from the RTC 4, an A/D converter 31 which converts an analog signal output from the sensor 6 into a digital signal, a serial communication interface (SCI) 36 which transmits and receives serial signals to and from the wireless communication unit 2, a parallel interface (PIO) 37 which controls the wireless communication unit 2 and the switch 8, and an oscillation unit (OSC) 30 which supplies the respective units in the microcomputer 3 with clocks. The respective units in the microcomputer 3 are coupled with each other via a system bus 38. The RTC 4 outputs interrupt signals (timer interrupts) in a given cycle, which is set in advance, to the interrupt control unit 35 of the microcomputer 3, and outputs reference clocks to the SCI 36. The PIO 37 controls the turning on/off of the switch 8 in accordance with a command from the CPU 34 to thereby control power supply to the sensor 6.

The bracelet type sensor node 1 starts up the microcomputer 3 in a given sampling cycle (for example, a 0.05-second cycle) to obtain sensing data from the sensor 6, and attaches an identifier for identifying the bracelet type sensor node 1 as well as a time stamp to the obtained sensing data before transmitting the sensing data to the base station 102. Details of the control of the bracelet type sensor node 1 may be as described in JP 2008-59058 A, for example. The bracelet type sensor node 1 may periodically transmit to the base station 102 sensing data that is obtained in a continuous manner.

<Outline of the System>

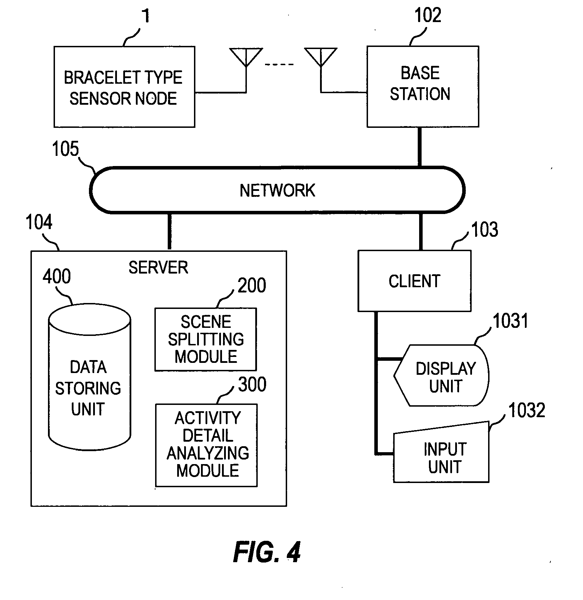

FIG. 4 is a block diagram illustrating function elements of the life log system of FIG. 1. Sensing data transmitted by the bracelet type sensor node 1 is received by the base station 102 and accumulated in a data storing unit 400 of the server 104 via the network 105.

The server 104 includes a processor, a memory, and a storage unit (which are not shown), and executes a scene splitting module 200 and an activity detail analyzing module 300. The scene splitting module 200 analyzes sensing data which contains the acceleration of the user's arm, and extracts individual actions as scenes based on a time-series transition in acceleration. The activity detail analyzing module 300 assigns action details to the extracted scenes, and presents concrete activity detail candidates that are associated with the respective action details on the client computer 103 of the user. The client computer 103 includes a display unit 1031 and an input unit 1032. The server 104 stores, as a life log, in the data storing unit 400, data in which action details or activity details are assigned to an extracted scene. The data storing unit 400 stores sensing data to which the identifier of the bracelet type sensor node 1 is attached. Each user is identified by attaching an identifier for identifying the user (for example, the identifier of the bracelet type sensor node 1) to the user's life log.

The scene splitting module 200 and the activity detail analyzing module 300 are, for example, programs stored in the storage unit (recording medium) to be loaded onto the memory at given timing and executed by the processor. Discussed below is an example in which the server 104 executes the scene splitting module 200 and the activity detail analyzing module 300 in a given cycle (for example, a twenty-four-hour cycle).

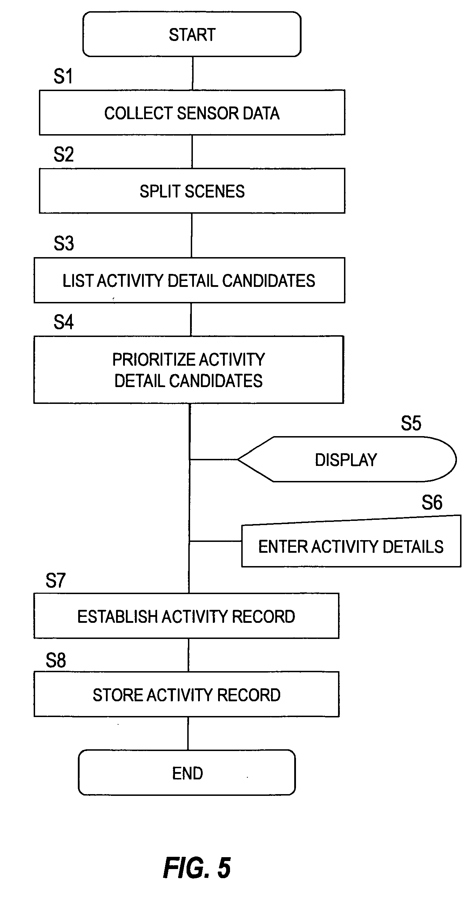

FIG. 5 is a flow chart illustrating the overall flow of processing that is executed in the life log system. First, in Step S1, sensing data transmitted from the bracelet type sensor nodes 1 is transferred by the base station 102 to the server 104, where the sensing data is accumulated in the data storing unit 400.

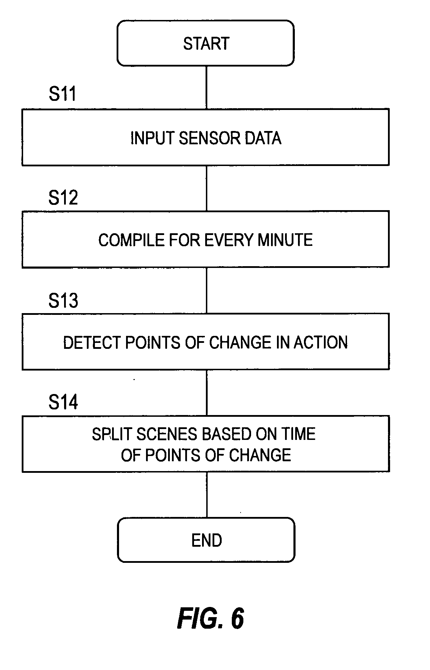

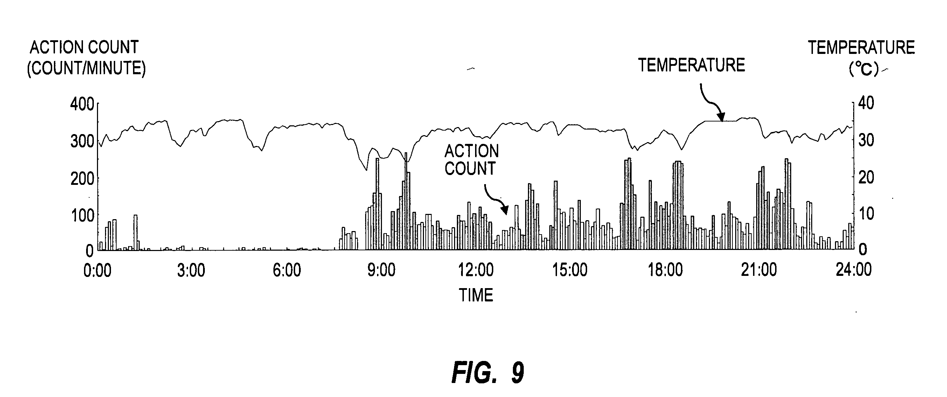

Next, in Step S2, the server 104 executes the scene splitting module 200 in a given cycle to extract a series of action states of a user as a scene from the sensing data accumulated in the data storing unit 400. The processing of the sensing data accumulated in the data storing unit 400 is executed by the scene splitting module 200 for each user (for each identifier that identifies one of the bracelet type sensor nodes 1). The scene splitting module 200 of the server 104 calculates the user's action count per unit time (for example, one minute) from time-series sensing data on acceleration, in a manner described later with reference to FIG. 6 and other drawings. Results of the action count calculation are data in which the action counts per unit time are sorted in time series as illustrated in FIG. 9. Next, the scene splitting module 200 extracts, as one scene, a period in which the user is inferred to be in the same action state from the obtained time-series action counts.

Specifically, the scene splitting module 200 extracts time-series points of change in action count per unit time, and extracts a period from one point of change to the next point of change as a scene in which the user is in the same action state. A point of change in action count is, for example, a time point at which a switch from a heavy exertion state to a calm state occurs. In extracting a scene, this invention focuses on two action states, sleeping and walking, which is a feature of this invention. For example, a person wakes up in the morning, dresses himself/herself, and goes to work. During work hours, the person works at his/her desk, moves to a conference room for a meeting, and goes to the cafeteria to eat lunch. After work, the person goes home, lounges around the house, and goes to sleep. Thus, in general, a day's activities of a person are roughly classified into waking and sleeping. Further, activities during waking hours often include a repetition of moving by walking before doing some action, completing the action, and then moving by walking before doing the next action. In short, daily activity scenes of a person can be extracted by detecting sleeping and walking. Through the processing described above, the scene splitting module 200 extracts scenes in a given cycle and holds the extracted scenes in the data storing unit 400.

Next, in Step S3, the server 104 processes each scene within a given cycle that has been extracted by the scene splitting module 200 by estimating details of actions done by the user based on the user's action count, and setting the estimated action details to the scene. The activity detail analyzing module 300 uses given determination rules, which is to be described later, to determine action details from a combination of the action count in data compiled for every minute, sleeping detection results and walking detection results, and assigns the determined action details to the respective scenes. Determining action details means, for example, determining which one of "sleeping", "resting", "light work", "walking", "jogging", and "other exercises" fits the action details in question.

In activity detail candidate listing processing (Step S3), the activity detail analyzing module 300 executes pre-processing in which segmentalized scenes are combined into a continuous scene. Specifically, when the user's sleep is constituted of a plurality of scenes as described below, the activity detail analyzing module 300 combines the nearest sleeping scenes into one whole sleeping scene. For instance, in the case where the user temporarily gets up after he/she went to bed in order to go to a bathroom or the like, and then goes back to sleep, the plurality of sleeping scenes can be regarded as one sleeping scene in the context of a day's activity pattern of a person. The activity detail analyzing module 300 therefore combines the plurality of sleeping scenes into one sleeping scene. To give another example, walking may include a resting scene such as waiting for a traffic light to change. In such cases, if a resting scene included in a period of walking, for example, from home to a station, satisfies a condition that the length of resting period is less than a given value, the activity detail analyzing module 300 combines walking scenes that precedes and follows the resting period into one whole walking scene.

Through the processing up through Step S3, scenes are assigned to all time periods and action details are assigned to the respective scenes.

In the subsequent Step S4, the activity detail analyzing module 300 performs activity detail candidate prioritizing processing for each set of action details in order to enter a more detailed account of activities done by the user who is wearing the bracelet type sensor node 1. The activity detail candidate prioritizing processing involves applying pre-registered rules to the action details assigned to the scene in order to determine the pattern of the action details, and generating candidates for concrete details of the user's activity. The concrete activity detail candidates are treated as candidates for finer activity details to be presented to the user in processing that is described later.

The pre-registered rules are specific to each user, and are rules for determining concrete activity detail candidates which use the combination of a single scene, or a plurality of scenes, and action details, and the time (s) of the scene(s). For example, in the case of action details "walking early in the morning", "strolling" can be determined as one of concrete activity detail candidates. To give another example, "walking (for 10 to 15 minutes), resting (for 20 to 25 minutes), and walking (for 7 to 10 minutes) that occur in 30 to 90 minutes after waking up" is determined as "commuting", which is a regular pattern in the usual life of that particular user. While a set of activity details corresponds to a combination of action details and accordingly constituted of a plurality of scenes in many cases, some activity details are defined by a single set of action details and a time as in the case of strolling mentioned above.

Next, the activity detail analyzing module 300 prioritizes activity detail candidates selected in accordance with the determination rules described above, in order to present the activity detail candidates in descending order of likelihood of matching details of the user's activity, instead of in the order in which the activity detail candidates have been selected.

In Step S5, the server 104 presents concrete activity detail candidates of each scene in the order of priority on the client computer 103. In Step S6, the user operating the client computer 103 checks activity details that are associated with the scene extracted by the server 104, and chooses from the activity details presented in the order of priority. The user can thus create a daily life log with ease by simply choosing the actual activity details from likely activity details.

In Step S7, the activity detail analyzing module 300 sets activity details chosen on the client computer 103 to the respective scenes to establish a life log (activity record).

The thus created life log is stored in Step S8 in the data storing unit 400 of the server 104 along with the identifier of the user and a time stamp such as the date/time of creation.

In this manner, a series of action states is extracted as a scene from sensing data, and action details are identified for each scene based on the action count in the scene. Activity details are then estimated from the appearance order of the action details, and the estimated activity detail candidates are presented to the user. This makes it easy for the user to enter activity details of each scene, and lessens the burden of creating an activity history.

The life log system of this invention has now been outlined. Described below are details of the system's components.

<Scene Splitting Module>

FIG. 6 is a flow chart illustrating an example of the processing that is executed by the scene splitting module 200 of the server 104. First, the scene splitting module 200 reads sensing data out of the data storing unit 400 for each identifier assigned to one of the bracelet type sensor nodes 1 in association with the identifier of a user of the life log system (Step S11). In this step, the scene splitting module 200 reads sensing data measured during, for example, a given cycle (e.g., twenty-four hours) which is a sensing data analysis cycle.

Next, in Step S12, a feature quantity of each given time interval (e.g., one minute) is calculated for acceleration data of the sensing data read by the scene splitting module 200. The feature quantity used in this embodiment is a zero cross count that indicates the action count of the wearer (user) of the bracelet type sensor node 1 within a given time interval.

Sensing data detected by the bracelet type sensor node 1 contains acceleration data of the X, Y, and Z axes. The scene splitting module 200 calculates the scalar of acceleration along the three axes, X, Y, and Z, calculates as the zero cross count the number of times the scalar passes 0 or a given value in the vicinity of 0, calculates the zero cross count within the given time interval (i.e., a frequency at which a zero cross point appears within the given time interval), and outputs this appearance frequency as the action count within the given time interval (e.g., one minute).

When Xg, Yg, and Zg are given as the acceleration along the respective axes, the scalar is obtained by the following expression:

$$\text{Scalar} = (Xg^2 + Yg^2 + Zg^2)^{1/2}$$

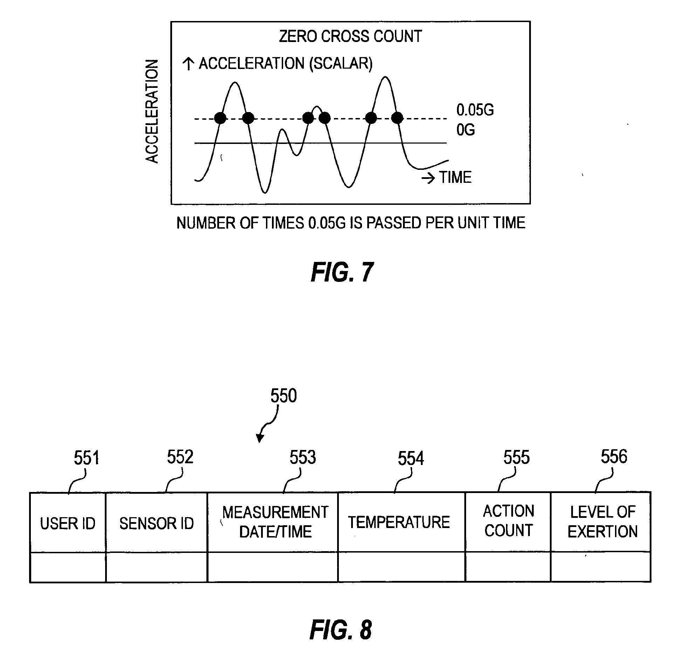

The scene splitting module 200 next performs filtering (band pass filtering) on the obtained scalar to extract only a given frequency band (for example, 1 Hz to 5 Hz) and remove noise components. The scene splitting module 200 then calculates, as illustrated in FIG. 7, as the zero cross count, the number of times the filtered scalar of the acceleration reaches a given threshold (for example, 0 G or 0.05 G. The threshold in the example of FIG. 7 is 0.05 G). Alternatively, the scene splitting module 200 calculates as the zero cross count the number of times the scalar of the acceleration crosses a given threshold. The zero cross count within the given time interval is then obtained as the action count. The scene splitting module 200 also obtains the integral value of the amount of exertion within the given time interval from the zero cross count and the scalar as level of exertion. The scene splitting module 200 further obtains the average temperature within the given time interval from the temperature contained in the sensing data.

Obtaining the zero cross count as the number of times a value in the vicinity of the threshold 0 G is crossed, instead of the number of times 0 G is crossed, prevents erroneous measurement due to minute vibrations that are not made by an action of a person, or due to electrical noise.

The scene splitting module 200 obtains the action count, the average temperature, and the level of exertion for each given time interval to generate data compiled for each given time interval as illustrated in FIG. 8, and accumulates the data in the data storing unit 400. FIG. 8 is an explanatory diagram illustrating the format of compiled data 550 compiled for each given time interval. In FIG. 8, each single entry of the compiled data 550 includes: a field for a sensor ID 552 which stores the identifier of the bracelet type sensor node 1 that is contained in sensing data; a field for a user ID 551 which stores the identifier of the wearer of the bracelet type sensor node 1 (a user of the life log system); a field for a measurement date/time 553 which stores the start time (measurement date/time) of the given time interval in question; a field for a temperature 554 which stores the temperature contained in the sensing data; a field for an action count 555 which stores an action count calculated by the scene splitting module 200; and a field for an level of exertion 556 which stores an level of exertion obtained by the scene splitting module 200. The compiled data 550 stores the identifier of the user in addition to the identifier of the bracelet type sensor node 1 because, in some cases, one person uses a plurality of bracelet type sensor nodes at the same time or uses different bracelet type sensor nodes on different occasions, and data of one node needs to be stored separately from data of another node in such cases.

As a result of the processing of Step S12, the scene splitting module 200 generates data compiled for each given time interval (e.g., one minute) with respect to a given cycle (e.g., twenty-four hours).

Next, in Step S13, the scene splitting module 200 compares the action count of the data compiled for one given time interval of interest against the action counts of data compiled respectively for the preceding and following time intervals. In the case where the difference in action count between the one time interval and its preceding or following time interval exceeds a given value, a time point at the border between these time intervals is detected as a point at which a change occurred in the action state of the wearer of the bracelet type sensor node 1, namely, a point of change in action.

In Step S14, a period between points of change in action detected by the scene splitting module 200 is extracted as a scene in which the user's action remains the same. In other words, the scene splitting module 200 deems a period in which the value of the action count is within a given range as a period in which the same action state is maintained, and extracts this period as a scene.

Through the processing described above, the scene splitting module 200 obtains the action count for each given time interval from sensing data detected within a given cycle, and extracts a scene based on points of change in action at which the action count changes.

<Activity Detail Analyzing Module>

An example of the processing of the activity detail analyzing module 300 is given below. For each scene within a given cycle that is extracted by the scene splitting module 200, the activity detail analyzing module 300 estimates details of an action made by the user based on the action count, and sets the estimated action details to the scene. The activity detail analyzing module 300 also presents concrete activity detail candidates of each scene.

As illustrated in FIG. 5 described above, the processing executed by the activity detail analyzing module 300 includes: processing of setting details of the user's action to each scene based on the compiled data generated for each time interval by the scene splitting module 200 (Step S3); processing of prioritizing candidates for details of the user's activity for each scene (Step S4); processing of presenting activity detail candidates of each scene on the client computer 103 (Step S5); processing of receiving selected activity detail candidates from the client computer 103 (Step S6); processing of generating an activity history by setting the received activity details to the respective scenes (Step S7); and processing of storing the activity history in the data storing unit 400 (Step S8).

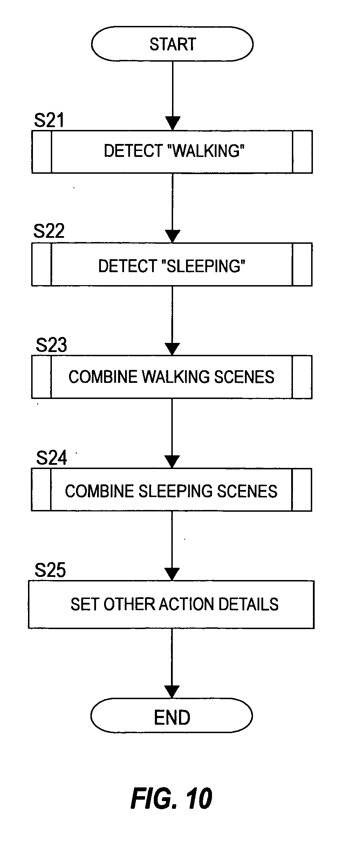

FIG. 10 is a flow chart illustrating an example of the processing of setting details of the user's action to each scene (Step S3). First, in Step S21, the activity detail analyzing module 300 extracts walking state scenes based on the action count of each given time interval. According to a method of detecting a walking state from the acceleration of the bracelet type sensor node 1 worn on the user's arm, waveforms observed include a cyclic change in the acceleration in the up-down direction (this change corresponds to the user's foot touching the ground on each step), regular repetition of the acceleration in the front-back direction in synchronization with the acceleration in the up-down direction (this repetition corresponds to a change in speed that occurs each time the user steps on the ground), and regular repetition of the acceleration in the left-right direction in synchronization with the acceleration in the up-down direction (this repetition corresponds to the user's body swinging to left and right on each step), and waveforms in which the swinging of the user's arms are added to the listed waveforms are observed as well. Based on those waveforms, whether a scene in question is a walking state or not can be determined. Alternatively, the reciprocal of the zero cross cycle may be detected as a step count. Those methods of detecting a walking state from an acceleration sensor worn on the human body can be known methods, an example of which is found in "Analysis of Human Walking/ Running Motion with the Use of an Acceleration/Angular Velocity Sensor Worn on an Arm" (written by Ko, Shinshu University Graduate School, URL http://laputa.cs.shinshu-u.ac.jp/-yizawa/ research/ h 16/koi.pdf).

Through the processing described above, "walking" is set as the action details of a scene determined as a walking state in Step S21.

In Step S22, the activity detail analyzing module 300 extracts sleeping scenes based on the action count. The action count in a sleeping state is very low, but is not zero because the human body moves in sleep by turning or the like. There are several known methods of identifying a sleeping state. For example, Cole's algorithm (Cole RJ, Kripke DF, Gruen W, Mullaney DJ, Gillin JC, "Automatic Sleep/Wake Identification from Wrist Activity", Sleep 1992, 15, 491-469) can be applied. The activity detail analyzing module 300 sets "sleeping" as the action details of a scene that is determined as a sleeping state by these methods.

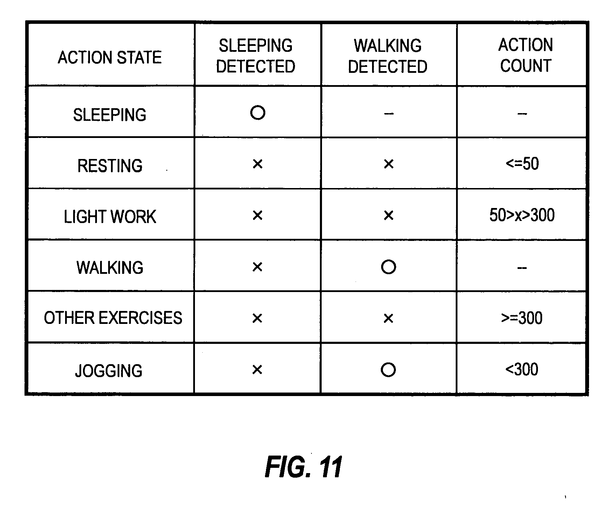

In Step S23, the activity detail analyzing module 300 refers to a determination value table illustrated in FIG. 11 in order to compare the action count of a scene that is neither the walking state nor the sleeping state against the determination values of "resting", "light work", "jogging", and "other exercises", and to determine which of the determination values the action count matches. The activity detail analyzing module 300 sets the result of the determination as the action details of the scene. FIG. 11 illustrates an example of the table in which determination values for determining action details are stored. The table is set in advance.

After setting action details to each scene within a given cycle in the manner described above, the activity detail analyzing module 300 executes Step S24 to select a plurality of scenes with "walking" set as their action details and sandwiching other action states such as "resting", and to combine the scenes into one walking scene. As mentioned above, because the action of walking is sometimes stopped temporarily by waiting for a traffic light to change, the use of an escalator or an elevator, or the like, simply splitting scenes does not yield a continuous walking scene. By combining scenes into one walking scene, a scene in which walking ceased temporarily can be understood as a form of a walking state in the viewing of a day's activity history of the user.

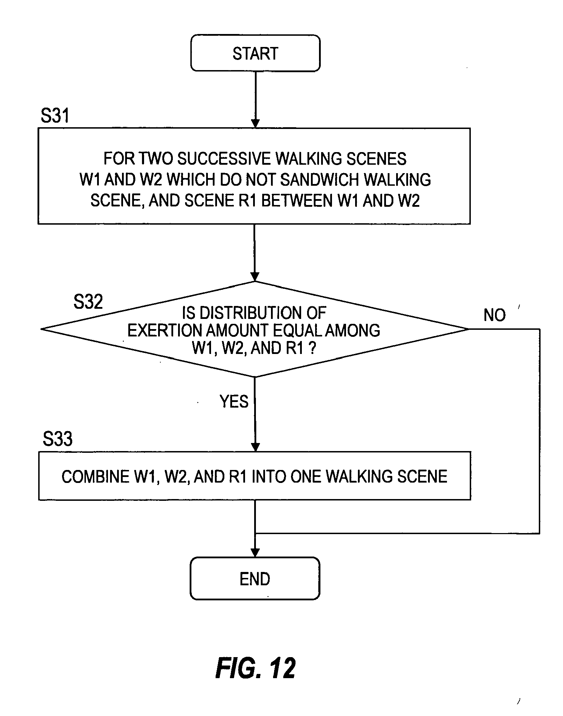

The processing of combining walking scenes is executed as illustrated in the flow chart of FIG. 12. First, in Step S31, the activity detail analyzing module 300 picks up a walking scene W1 and, in the case where a scene R1 which follows the walking scene W1 is other than "walking" and is followed by a walking scene W2, starts this processing.

In Step S32, the activity detail analyzing module 300 compares the amounts of exertion of the three successive scenes, W1, R1, and W2. In the case where these amounts of exertion are distributed equally, the activity detail analyzing module 300 proceeds to Step S33, where the three scenes, W1, R1, and W2, are combined into one walking scene W1. Specifically, the activity detail analyzing module 300 changes the end time of the scene W1 to the end time of the scene W2, and deletes the scenes R1 and W2. The activity detail analyzing module 300 may instead change the action details of the scene R1 to "walking" to combine the plurality of scenes.

In evaluating how the amount of exertion is distributed, the distribution of the amount of exertion in R1 and the distribution of the amount of exertion in W1 or W2 may be determined as equal when, for example, the ratio of the average action count in R1 to the average action count in one of W1 and W2 is within a given range (e.g., within ±20%).

Alternatively, for instance, when the action count of the scene R1 is very low but the length of the scene R1 is within a given length of time (e.g., a few minutes), the three scenes may be combined into one walking scene.

Next, in Step S25 of FIG. 10, the activity detail analyzing module 300 selects a plurality of scenes with "sleeping" set as their action details and sandwiching other action states such as "walking", and combines the scenes into one sleeping scene.

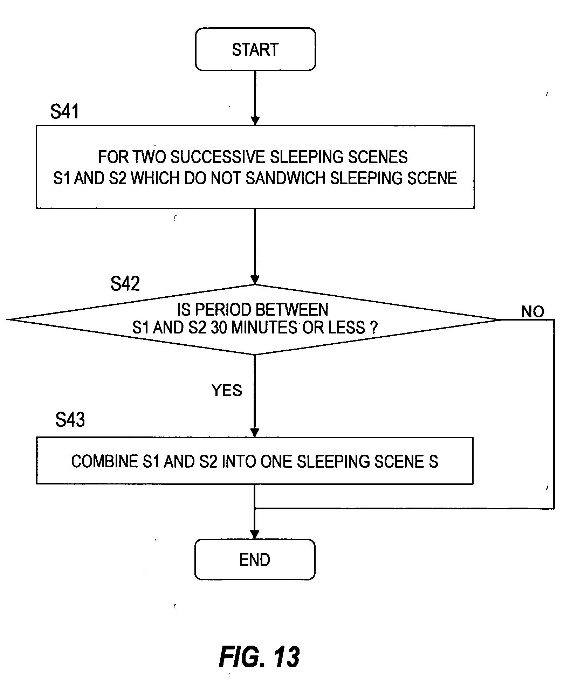

The processing of combining sleeping scenes is executed as illustrated in the flow chart of FIG. 13. First, in Step S41, the activity detail analyzing module 300 picks up a sleeping scene S1 and, in the case where a scene R2 which follows the sleeping scene S1 is other than "sleeping" and is followed by a sleeping scene S2, starts this processing.

In Step S42, the activity detail analyzing module 300 examines the three successive scenes and, in the case where a period from the end time of the scene S1 and the start time of the scene S2 is equal to or less than a given length of time (e.g., 30 minutes), proceeds to Step S43, where the three scenes, S1, R2, and S2, are combined into one sleeping scene S1. Specifically, the activity detail analyzing module 300 changes the end time of the scene S1 to the end time of the scene S2, and deletes the scenes R2 and S2.

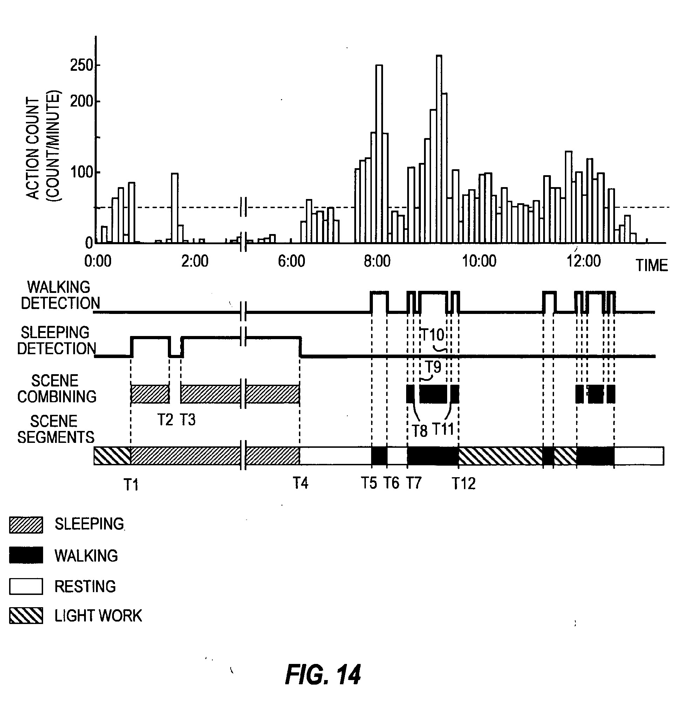

Through the processing described above, the activity detail analyzing module 300 sets preset action details to each scene generated by the scene splitting module 200 and, in the case of walking scenes and sleeping scenes, combines a plurality of scenes that satisfies a given condition into one scene to simplify scenes that are split unnecessarily finely. As a result, as illustrated in FIG. 14, walking detection and sleeping detection are executed to respectively extract walking scenes and sleeping scenes based on the action count calculated for each given time interval by the scene splitting module 200, and then other action details than walking and sleeping are set to each remaining scene.

With action details set to each scene, sleeping scenes between times T1 and T4 illustrated in scene combining of FIG. 14 sandwich a period from time T2 to time T3 where the action is other than sleeping. In the case where the period from time T2 to time T3 is within a given length of time, the sleeping scene combining described above is executed to combine the series of sleeping scenes between times T1 and T4 into one sleeping scene as illustrated in scene segments of FIG. 14.

Similarly, walking scenes between times T7 and T12 sandwich a period from time T8 to time T9 and a period from time T10 to time T11 where the action is other than walking. In the case where the period from time T8 to time T9 and the period from time T10 to time T11 satisfy a given condition, the walking scene combining described above is executed to combine the series of walking scenes between times T7 and T12 into one walking scene as illustrated in the scene segments of FIG. 14. It should be noted that a period from time T5 to time T6 is one sleeping scene.

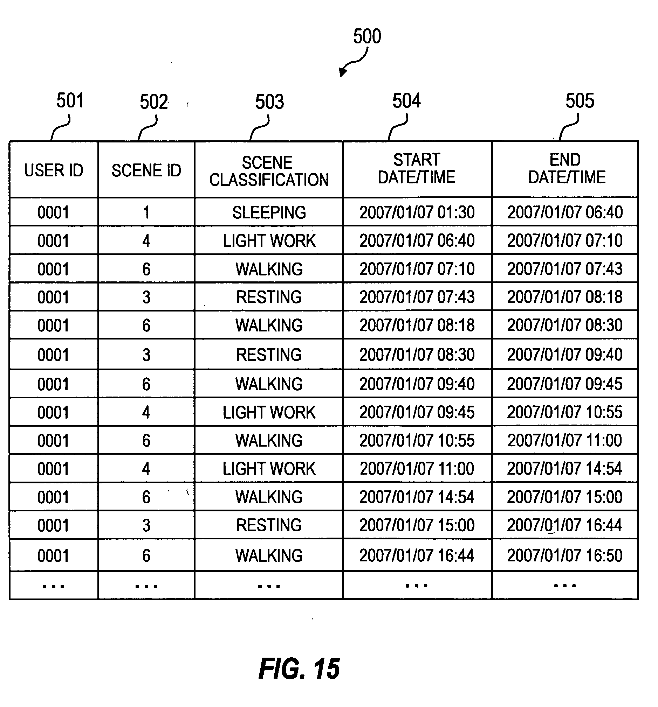

FIG. 15 is an explanatory diagram illustrating an example of scenes 500 (hereinafter, referred to as scene data) containing action details, which is generated by the activity detail analyzing module 300 as a result of the processing of FIG. 10. Each single entry of the scene data 500 includes: a field for a user ID 501 which indicates the identifier of a user; a field for a scene ID 502 which indicates an identifier assigned to each scene; a field for a scene classification 503 which stores action details assigned by the activity detail analyzing module 300; a field for a start date/time 504 which stores the start date and time of the scene in question; and a field for an end date/time 505 which stores the end date and time of the scene.

Next, the activity detail analyzing module 300 prioritizes candidates for details of the user's activity for each scene in order to present the details of the user's activity in addition to the assigned action details of the scene data 500. This is because, while action states of the user of the bracelet type sensor node 1 are split into scenes and preset action details are assigned to each scene in the scene data 500, expressing the user's activities (life) by these action details can be difficult. The activity detail analyzing module 300 therefore estimates candidates for activity details for each scene, prioritizes the sets of estimated activity details, and then presents these activity details for the selection by the user, thus constructing an activity history that reflects details of the user's activity.

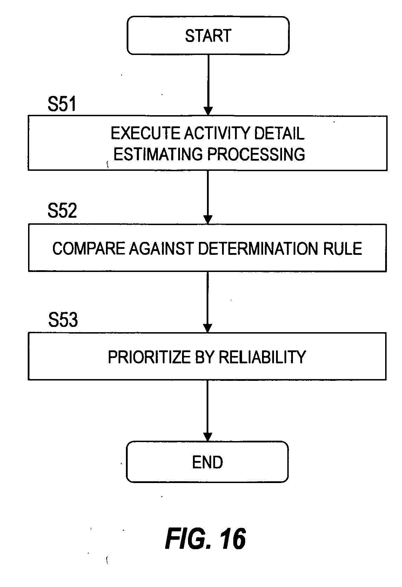

FIG. 16 is a flow chart illustrating an example of the processing executed by the activity detail analyzing module 300 to generate and prioritize activity detail candidates.

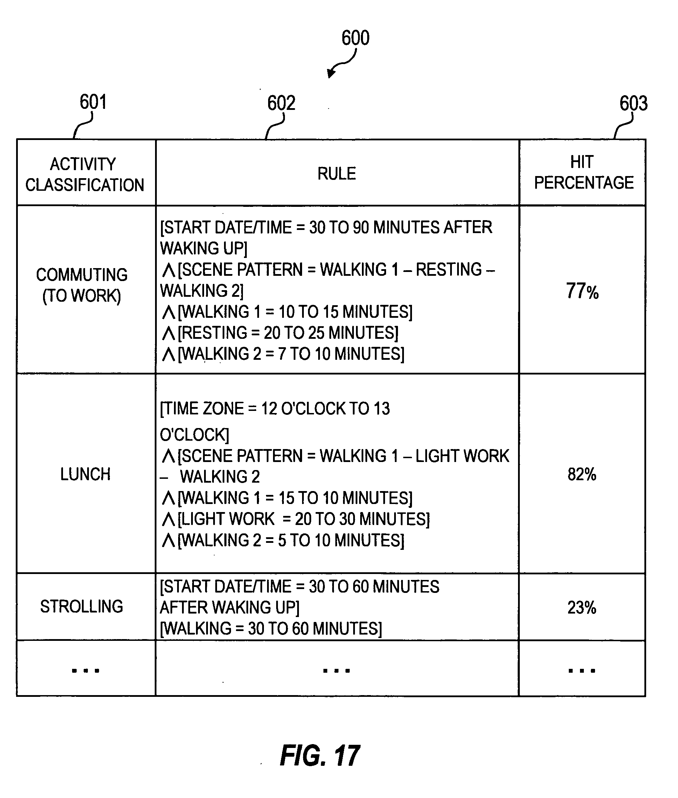

In Step S51, the activity detail analyzing module 300 reads the generated scene data 500, searches for a combination of scenes that matches one of scene determining rules, which are set in advance, and estimates activity details to be presented. The scene determining rules are specific to each user and define activity details in association with a single scene or a combination of scenes, the length of time or start time of each scene, and the like. The scene determining rules are set as illustrated in a scene determining rule table 600 of FIG. 17.

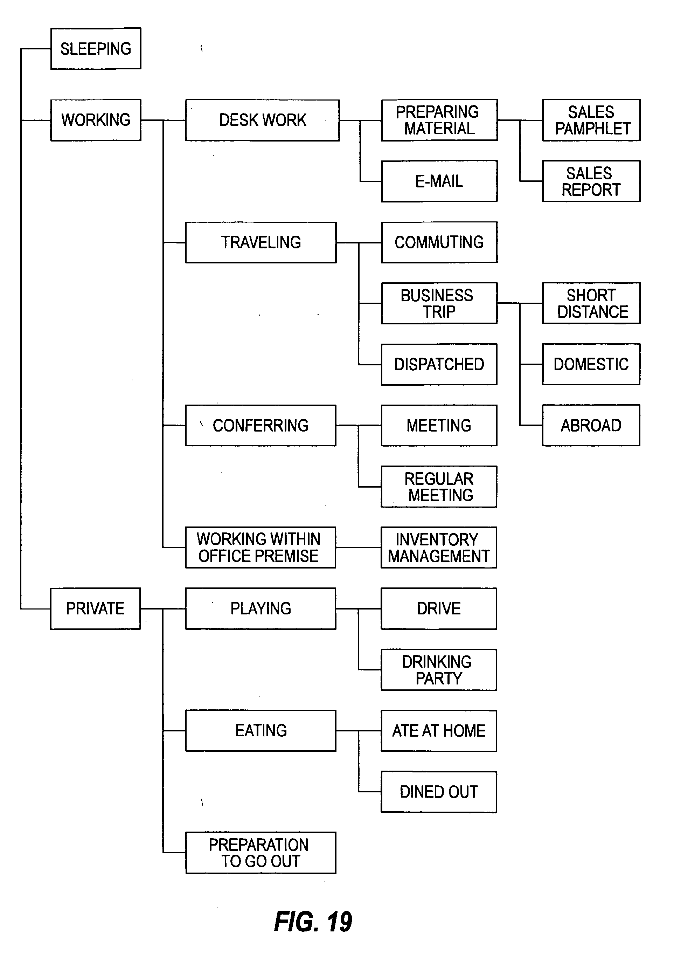

FIG. 17 is an explanatory diagram illustrating an example of the scene determining rule table 600. Each single entry of the scene determining rule table 600 includes: a field for an activity classification 601 which stores activity details; a field for a rule 602 in which a scene pattern, a start time or a time zone, and the lengths of the scenes are defined in association with the activity details; and a field for a hit percentage 603 which stores a rate at which the activity details was actually chosen by the user when presented on the client computer 103 by the activity detail analyzing module 300. Activity details of the activity classification 601 of the scene determining rule table 600 are kept in the data storing unit 400 of the server 104 in the form of tree structure data of FIG. 19 as an activity detail item management table 900. The rule 602 can be set for each set of activity details.

The activity detail analyzing module 300 refers to scenes contained in the generated scene data 500 in order from the top, and extracts a single scene or a combination of scenes that matches one of scene patterns stored as the rule 602 in the scene determining rule table 600.

Next, in Step S52, the activity detail analyzing module 300 compares the lengths of time and times of the scenes extracted from the scene data 500 against the lengths of time and times of the respective scenes in the rule 602, and extracts a combination of the extracted scenes of the scene data 500 that matches the rule 602. Activity details stored as the activity classification 601 in association with this rule 602 are set as a candidate for the extracted scenes of the scene data 500. For instance, a scene in the scene data 500 to which "walking" is set as action details is picked up and, in the case where its next scene is "resting" and its next-to-next scene is "walking", the combination of these three scenes is associated with "commuting" of the activity classification 601 as an activity detail candidate. To achieve this, the activity detail analyzing module 300 compares the start dates/times and the lengths of time of the respective scenes in the rule 602 with the times and lengths of time of the respective scenes of the scene data 500. When the times and lengths of time of the respective scenes of the scene data 500 satisfy the condition of the rule 602, the activity detail analyzing module 300 sets "commuting" as a candidate for activity details of the three scenes of the scene data 500.

In Step S53, the activity detail analyzing module 300 calculates as the percentage of hits a rate at which the activity classification 601 extracted in Step S52 is actually chosen by the user. This rate can be calculated from the ratio of a frequency at which the extracted activity classification 601 has been chosen by the user to a frequency at which the extracted activity classification 601 has been presented. In the case where a plurality of activities stored as the activity classification 601 is associated with the extracted scenes of the scene data 500, the activity detail analyzing module 300 sorts these activities stored as the activity classification 601 by the percentage of hits.

Through the processing of Steps S51 to S53, each entry of the scene data 500 generated by the scene splitting module 200 is compared against scene patterns, and activity detail candidates associated with a combination of scenes of the scene data 500 are extracted and sorted by the percentage of hits.

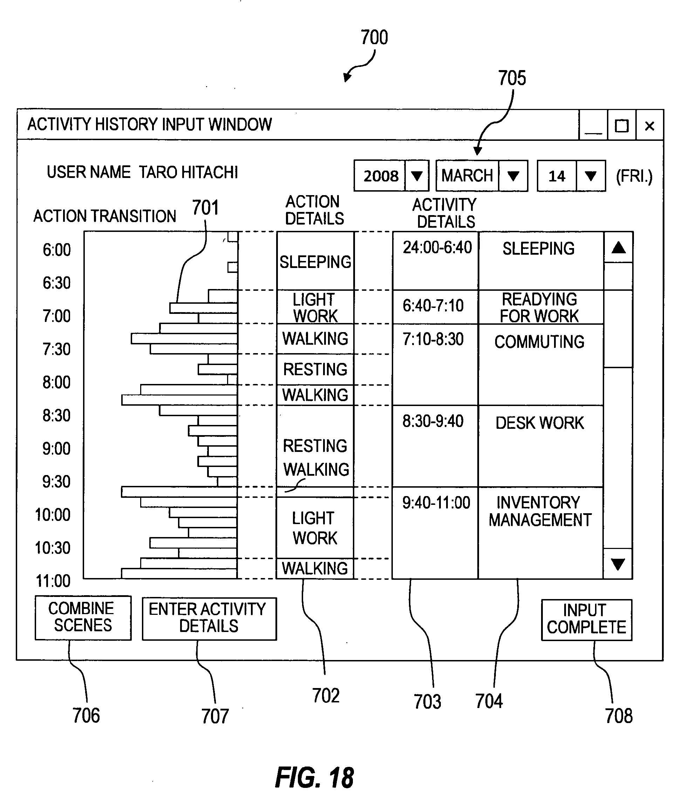

Next, the server 104 receives from the client computer 103 a request to input an activity history, and displays an activity history input window 700 illustrated in FIG. 18 on the display unit 1031 of the client computer 103.

FIG. 18 is a screen image of the activity history input window 700 displayed on the display unit 1031 of the client computer 103. The server 104 receives a user ID and other information from the client computer 103, and displays the compiled data 550, the scene data 500, and activity detail candidates of the specified user in the activity history input window 700. A browser can be employed as an application run on the client computer 103.

The activity history input window 700 includes an action count 701, action details 702, a time display 703, activity details 704, a date/time pulldown menu 705, a "combine scenes" button 706, an "enter activity details" button 707, and an "input complete" button 708. The action count 701 takes the form of a bar graph in which the values of the action count 555 are displayed in relation to the values of the measurement date/time 553 in the compiled data 550. As the action details 702, action details stored as the scene classification 503 in the scene data 500 are displayed. The time display 703 displays the start date/time 504 and the end date/time 505 in the scene data 500. In a field for the activity details 704, activity details are entered or displayed. The date/time pulldown menu 705 is used to set the date and time when the activity history is entered. The "combine scenes" button 706 is used to send to the server 104 a command to manually combine a plurality of scenes. The "enter activity details" button 707 is used to enter the activity details 704 specified by the user with the use of a mouse cursor or the like. The "input complete" button 708 is used to command to complete the input. In the activity history input window 700 of the drawing, the input of the activity details 704 has been completed.

The user operating the client computer 103 selects the "enter activity details" button 707 and then selects the activity details 704 on the activity history input window 700, causing the activity history input window 700 to display activity detail candidates obtained by the activity detail analyzing module 300. The user operates a mouse or the like that constitutes a part of the input unit 1032 of the client computer 103 to choose from the activity detail candidates. In the case where the activity detail candidates do not include the desired item, the user may enter activity details manually. The user may also manually modify activity details chosen from among the activity detail candidates.

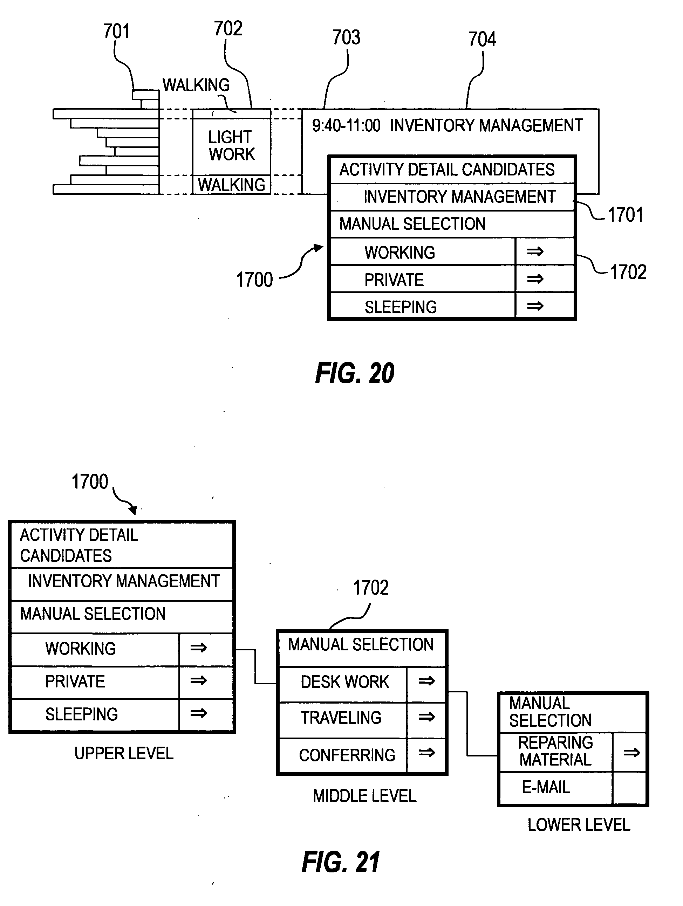

When the user selects the activity details 704, the server 104 displays in the field for the activity details 704 activity detail candidates estimated by the activity detail analyzing module 300 for each entry of the scene data 500. For example, when the user selects the activity details 704 that are associated with the "light work" scene started from 9:40 of FIG. 18, a candidate box 1700 containing activity detail candidates is displayed as illustrated in FIG. 20. The candidate box 1700 has two fields, for activity detail candidates 1701 estimated by the activity detail analyzing module 300, and for manual selection candidates 1702 selected manually from the activity detail item management table 900, which is set in advance. The user can enter finer activity details by selecting an item that is displayed in the candidate box 1700. When choosing activity details from the manual selection candidates 1702, the user can choose from activity details hierarchized in advance into upper level concepts, middle level concepts, and lower level concepts as illustrated in FIG. 21.

Once the user selects from candidates presented on the display unit 1031 of the client computer 103, the activity history of the selected scene data 500 is established. The server 104 generates the activity history and stores the activity history in an activity detail storing table 800 of the data storing unit 400. The server 104 also updates the percentage of hits for the activity detail candidates selected by the user.

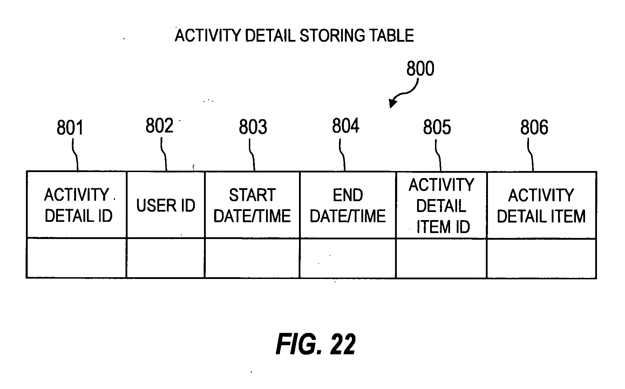

FIG. 22 is an explanatory diagram illustrating an example of the activity detail storing table 800 which stores an activity history. Each entry of the activity detail storing table 800 includes: a field for an activity detail ID 801 which stores the identifier of a set of activity details; a field for a user ID 802 which stores the identifier of a user; a field for a start date/time 803 which stores a time stamp indicating the date and time when the activity in question is started; a field for an end date/time 804 which stores a time stamp indicating the date and time when the activity in question is ended; a field for an activity detail item ID 805 which stores the identifier of activity details in a tree structure; and a field for an activity detail item ID 806 which stores the name of an activity detail item.

Activity detail items set by the user are thus stored as an activity history in the activity detail storing table 800 within the data storing unit 400 of the server 104, and can be referenced from the client computer 103 at any time.

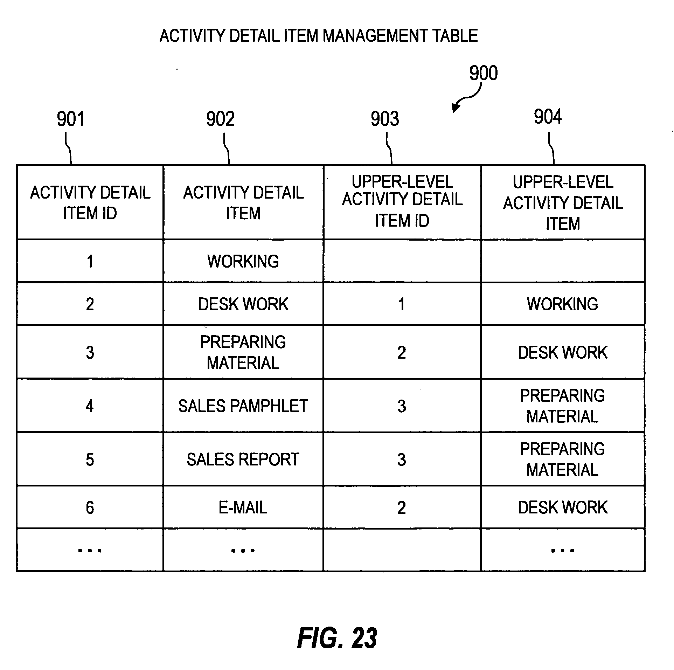

FIG. 23 is an explanatory diagram illustrating an example of the activity detail item management table 900 which stores the activity detail items of FIG. 19. The activity detail item management table 900 is kept in the data storing unit 400 of the server 104. Each single entry of the activity detail item management table 900 includes: a field for an activity detail item ID 901 which stores the identifier of an activity detail item; a field for an activity detail item 902 which stores the name of the activity detail item; a field for an upper-level activity detail item ID 903 which indicates the identifier of an upper-level activity detail item in the tree structure; and a field for an upper-level activity detail item 904 which stores the name of the upper-level activity detail item in the tree structure.

The activity detail item management table 900 has a hierarchical structure containing upper to lower level concepts of activity details. An activity is defined more concretely by using a lower level concept that is further down the hierarchy. This way, a user who intends to record his/her activities in detail can use a lower level concept to write a detailed activity history, and a user who does not particularly intend to keep a detailed record can use an upper level concept to enter an activity history. This enables users to adjust the granularity of input to suit the time or labor that can be spared to, or the willingness to, create an activity history, and thus prevents users from giving up on creating an activity history.

<Conclusion>

According to this invention, a user's day-to-day action state is measured by the acceleration sensor of the bracelet type sensor node 1 and stored on the server 104. The measured action state is analyzed in a given cycle, and scenes are automatically extracted from the user's day-to-day action state to generate the scene data 500. The server 104 automatically sets action details that indicate the details of the action to the scene data 500 generated automatically by the server 104. The user of the life log system can therefore recall details of the past activities with ease. The server 104 further estimates candidates for details of an activity done by the user based on action details of the respective scenes, and presents the candidates to the user. The user can create an activity history by merely selecting the name of an activity detail item from the presented candidates. This allows the user to enter an activity history with greatly reduced labor.

In the extracted scenes of the scene data 500, sleeping, walking, and other action states are distinguished clearly from one another to use sleeping and walking in separating one activity of a person from another activity. Candidates for activity details can thus be estimated easily.

In the life log system of this invention, scenes are assigned to all time periods within a given cycle, action details are assigned to the respective scenes, and then a combination of the scenes is compared against determination rules in the scene determining rule table 600 to estimate concrete activity detail candidates. An activity of a person is a combination of actions in most cases, and a single set of activity details often includes a plurality of scenes, though there indeed are cases where one scene defines one set of activity details (for instance, walking early in the morning is associated with activity details "strolling").

Accordingly, a combination of action details is defined as a scene pattern in the scene determining rule table 600, and compared with the appearance order of action details (scene classification 503) of the scene data 500, to thereby estimate activity detail candidates that match a scene. In the case of activity details "commuting", for example, action details "walking", "resting", and "walking" appear in order. Then scenes in which the same combination of action details as above appears in the same order as above are extracted from the scene data 500. The activity details of the extracted scenes of the scene data 500 can therefore be estimated as "commuting". The life log system further compares the times of the extracted scenes of the scene data 500 against times defined in the scene determining rule table 600, to thereby improve the accuracy of activity detail estimation.

For each candidate, the activity detail determining rule 602 keeps, as a hit percentage value based on the ratio of the past adoption and rejection, a rate at which the candidate was actually chosen when presented. Activity detail candidates are presented to the user in descending order of hit percentage, thereby presenting to the user the activity detail candidates in descending order of likelihood of being chosen by the user. While presenting all activity detail candidates is one option, only candidates that have a given hit percentage, which is determined in advance, or higher may be displayed, or only a given number of (e.g., five) candidates from the top in descending order of hit percentage may be displayed. This prevents the presentation from becoming complicated.

The embodiment described above deals with an example in which the acceleration sensor of the bracelet type sensor node 1 is used to detect the action state of a user (i.e., human body) of the life log system. However, any type of living organism information can be used as long as the action state of the human body can be detected. For example, pulse or step count may be used. Alternatively, a plurality of types of living organism information may be used in combination to detect the action state of the human body. Human body location information obtained via a GPS, a portable terminal, or the like may be used in addition to living organism information. Besides living organism information and location information, a log of a computer, a portable terminal, or the like that is operated by the user may be used to identify details of light work (for example, writing e-mail).

The sensor node used to detect living organism information is not limited to the bracelet type sensor node 1, and can be any sensor node as long as the sensor node is wearable on the human body.

The embodiment described above deals with an example in which scene patterns and activity details are set in advance in the scene determining rule table 600. Alternatively, the server 104 may learn the relation between activity details determined by the user and a plurality of scenes to set the learned relation in the scene determining rule table 600.

The embodiment described above deals with an example in which the server 104 and the client computer 103 are separate computers. Alternatively, the functions of the server 104 and the client computer 103 may be implemented by the same computer.

<Modification Example>

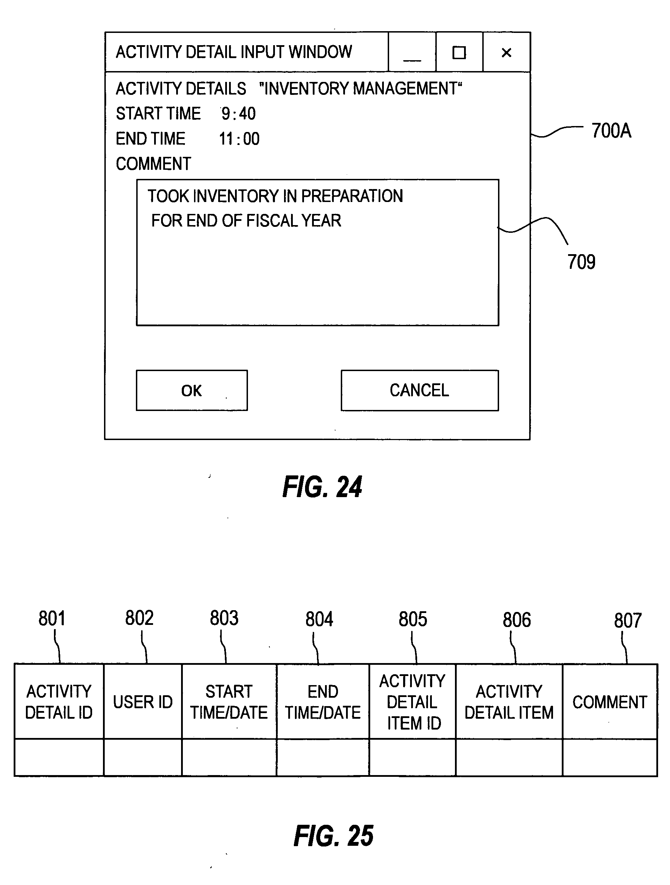

FIGS. 24 and 25 illustrate a first modification example of the embodiment of this invention. In the first modification example, a comment window for writing a note or the like is added to the activity history input window 700 of the embodiment described above.

FIG. 24 illustrates a comment input window 700A of the first modification example. The comment input window 700A of the first modification example has a comment field 709 in which text can be entered. The comment input window 700A pops up when, for example, the activity details 704 of FIG. 18 are operated by double clicking or the like, and receives text from the input unit 1032 of the client computer 103.

As illustrated in FIG. 25, a comment 807 is added to the activity detail storing table 800 which stores an activity history. Stored as the comment 807 is text in the comment field 709 that the server 104 receives from the client computer 103.

By supplying a detailed description in text through the comment field 709, a detailed activity history is created.

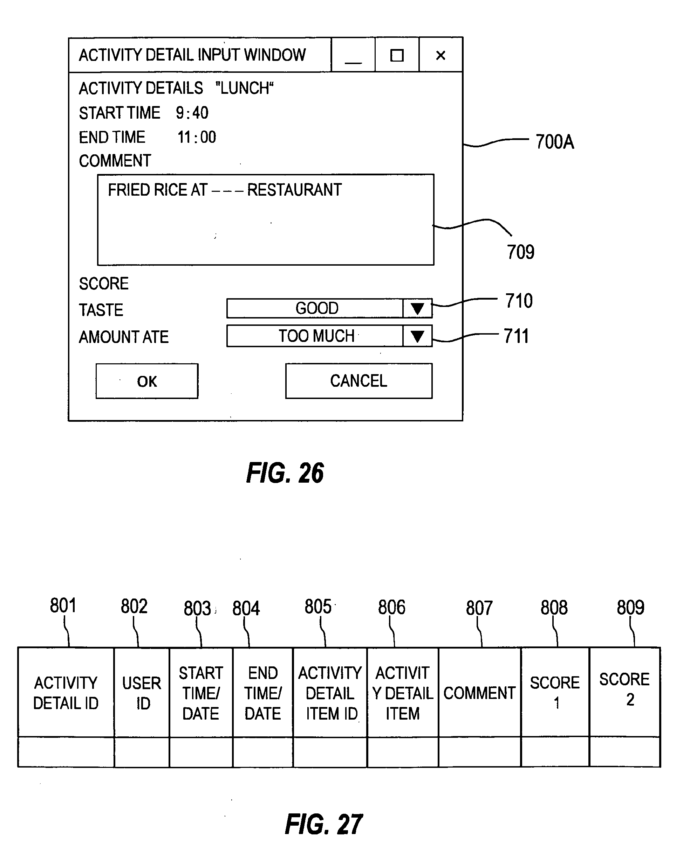

FIGS. 26 and 27 illustrate a second modification example of the embodiment of this invention. In the second modification example, an evaluation (score) can additionally be set to activity details on the comment input window 700A of the first modification example described above.

FIG. 26 illustrates the comment input window 700A of the second modification example. The comment input window 700A of the second modification example includes, in addition to the comment field 709 where text can be entered, a score 710 for storing a first evaluation and a score 711 for storing a second evaluation. The values of the scores 710 and 711 may be chosen from items set in advance.

As illustrated in FIG. 27, the scores 808 and 809 are added to the activity detail storing table 800 which stores an activity history. Stored as the scores 808 and 809 are the scores 710 and 711 that the server 104 receives from the client computer 103.

With the scores 710 and 711, evaluations on activity details can be added. For example, an evaluation on activity details "eating" is selected from "ate too much", "normal amount", and "less than normal amount", thus enabling users to create a more detailed activity history through simple operation.

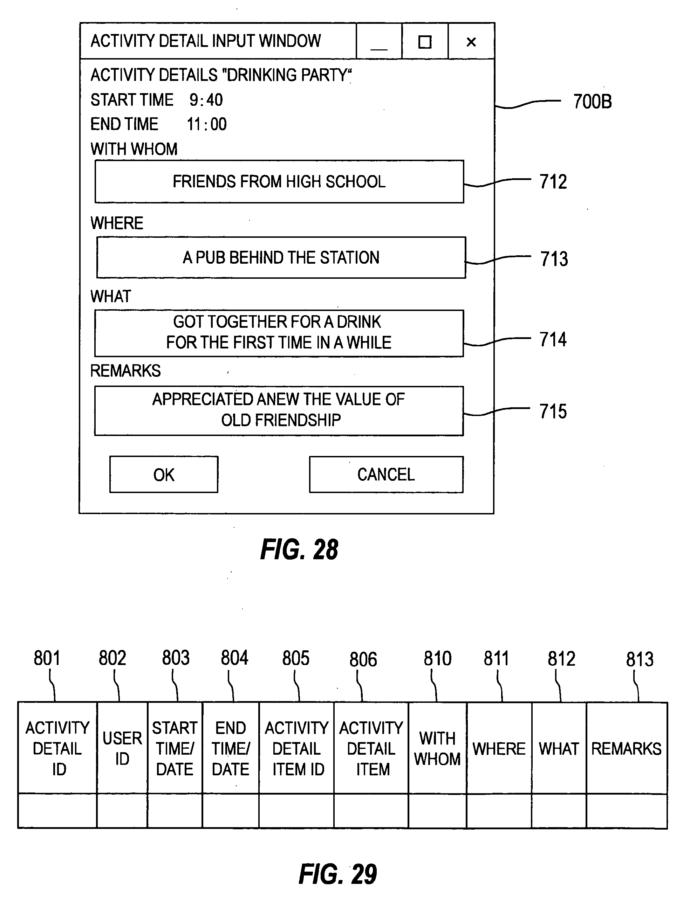

FIGS. 28 and 29 illustrate a third modification example of the embodiment of this invention. In the third modification example, additional information on activity details such as other participants of an activity can be written on the activity history input window 700 of the embodiment described above.

FIG. 28 illustrates an input window 700B of the third modification example. The input window 700B of the third modification example includes: a field for "with whom" 712 which can be used to enter in text a person's name associated with activity details in question or the like; a field for "where" 713 which can be used to enter in text a location associated with the activity details; a field for "what" 714 which can be used to enter finer details of the activity; and a field for "remarks" 715 which can be used to enter the user's thoughts on the activity details. The input window 700B pops up when, for example, the activity details 704 of FIG. 18 is operated by double clicking or the like, and receives text from the input unit 1032 of the client computer 103.

As illustrated in FIG. 29, "with whom" 810, "where" 811, "what" 812, and "remarks" 813 are added to the activity detail storing table 800 which stores an activity history. Stored as "with whom" 810, "where" 811, "what" 812, and "remarks" 813 are "with whom" 712, "where" 713, "what" 714, and "remarks" 715 that the server 104 receives in text from the client computer 103.

A more detailed activity history is created by adding a detailed description in text about participants and a location that are associated with activity details in question, and about the user's thoughts on the activity details.

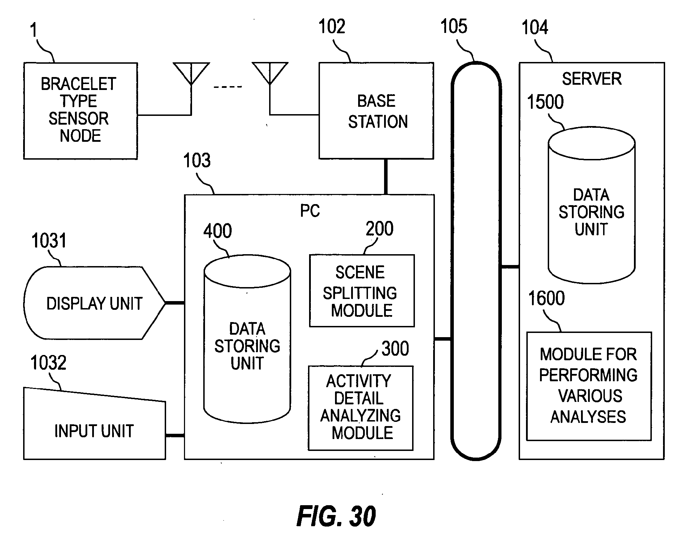

FIG. 30 illustrates a fourth modification example of the embodiment of this invention. The fourth modification example is the same as the embodiment described above, except that the system configuration of FIG. 4 is partially changed. In the fourth modification example, the client computer 103, instead of the server 104, includes the scene splitting module 200, the activity detail analyzing module 300, and the data storing unit 400. This client computer 103 is connected directly to the base station 102. The configurations of the scene splitting module 200, the activity detail analyzing module 300, and the data storing unit 400 are the same as in the embodiment described above. The client computer 103 is connected to the server 104 via the network 105. The server 104 includes a data storing unit 1500, which stores an activity history (the activity detail storing table 700) generated by and received from the client computer 103, and an analysis module 1600, which performs a given analysis on an activity history.

Industrial Applicability

As has been described, this invention is applicable to a computer system that automatically creates a person's activity history, and more particularly, to a sensor network system in which living organism information is transmitted to a server through wireless communication.

**Claims**

1. An activity history generating method of generating an activity history with a sensor, which is worn by a person to measure living organism information, and a computer, which obtains the living organism information from the sensor to identify an action state of the person, comprising the steps of:

   obtaining the living organism information by the computer and accumulating the living organism information on the computer;
   obtaining, by the computer, an action count from the accumulated living organism information;
   extracting, by the computer, a plurality of points of change in time series in the action count;
   extracting, by the computer, a period between the points of change as a scene in which the same action state is maintained;
   comparing, by the computer, the action count of each extracted scene against conditions set in advance to identify action details of the scene;
   estimating, by the computer, details of an activity that is done by the person during the scene based on an appearance order of the action details; and
   generating an activity history based on the estimated activity details.

2. The activity history generating method according to claim 1,
   wherein the step of estimating details of an activity that is done by the person during the scene based on an appearance order of the action details comprises the step of comparing preset relations between activity details and action detail appearance orders with the appearance order of the action details of the scene to estimate, as candidates for activity details of the scene, activity details to which the appearance order of the action details of the scene is a match, and
wherein the step of generating an activity history based on the estimated activity details comprises the steps of:

   choosing an activity detail candidate for the scene from among the estimated activity detail candidates; and
   generating an activity history that contains the chosen activity detail candidate as the activity details of the scene.

3. The activity history generating method according to claim 1, wherein the step of estimating details of an activity that is done by the person during the scene based on an appearance order of the action details comprises the steps of:

   comparing preset relations between activity details and action detail appearance orders with the appearance order of the action details of the scene to estimate, as candidates for activity details of the scene, activity details to which the appearance order of the action details of the scene is a match; and
   prioritizing the estimated activity detail candidates,

wherein the step of generating an activity history based on the estimated activity details comprises the steps of:

   choosing an activity detail candidate for the scene from among the estimated activity detail candidates in the order of priority; and
   generating an activity history that contains the chosen activity detail candidate as the activity details of the scene, and

wherein a place in the priority order is a value calculated based on a ratio of a frequency at which the estimated activity details become a candidate and a frequency at which the activity details are chosen as the activity history.

4. The activity history generating method according to claim 1, wherein the step of comparing, by the computer, the action count of each extracted scene against conditions set in advance to identify action details of the scene comprises the steps of:

   determining from the action count of the scene whether or not the action details of the scene are "walking";
   determining from the action count of the scene whether or not the action details of the scene are "sleeping"; and

when the action details of the scene are neither "walking" nor "sleeping", setting, to the scene, preset action details in accordance with a value of the action count of the scene.

5. The activity history generating method according to claim 1, further comprising the step of combining the scenes for which action details have been identified, wherein the step of combining the scenes for which action details have been identified comprises combining a first scene, a second scene, and a third scene which are successive in time series when the action details of the first scene and the action details of the third scene are the same and the second scene satisfies a given condition.

6. The activity history generating method according to claim 1, wherein the sensor comprises an acceleration sensor for detecting acceleration of an arm as the living organism information, and

wherein the step of obtaining, by the computer, an action count from the accumulated living organism information comprises obtaining a number of times the acceleration crosses a given threshold within a given time interval as the action count.

7. An activity history generating system, comprising:

a sensor worn by a person to measure living organism information;
a network for transferring the living organism information measured by the sensor to a computer; and
a computer which obtains the living organism information from the network to identify an action state of the person and, based on the action state, generates a history of activities done by the person,
wherein the computer comprises:

a data storing unit for accumulating the living organism information;
a scene splitting module for obtaining an action count from the living organism information accumulated in the data storing unit, and for obtaining a plurality of points of change in time series in the action count to extract a period between the points of change as a scene in which the same action state is maintained;
an activity detail analyzing module for comparing the action count of each extracted scene against conditions set in advance to identify action details of the scene, and for estimating details of an activity done by the person during the scene based on an appearance order of the action details; and
an activity history establishing module for generating an activity history based on the estimated activity details.

8. The activity history generating system according to claim 7, wherein the activity detail analyzing module compares the preset relations between activity details and action detail appearance orders with the appearance order of the action details of the scene to estimate, as candidates for activity details of the scene, activity details to which the appearance order of the action details of the scene is a match, and wherein the activity history establishing module receives a result of choosing an activity detail candidate for the scene from among the estimated activity detail candidates, and generates an activity history that contains the chosen activity detail candidate as the activity details of the scene.

9. The activity history generating system according to claim 7,
wherein the activity detail analyzing module compares the preset relations between activity details and action detail appearance orders with the appearance order of the action details of the scene to estimate, as candidates for activity details of the scene, activity details to which the appearance order of the action details of the scene is a match, and prioritizes the estimated activity detail candidates,
wherein the activity history establishing module receives a result of choosing an activity detail candidate for the scene from among the estimated activity detail candidates in the order of priority, and generates an activity history that contains the chosen activity detail candidate as the activity details of the scene, and
wherein a place in the priority order is a value calculated based on a ratio of a frequency at which the estimated activity details become a candidate and a frequency at which the activity details are chosen as the activity history.

10. The activity history generating system according to claim 7, wherein the activity detail analyzing module determines from the action count of the scene whether or not the action details of the scene are "walking" or "sleeping" and, when the action details of the scene are neither "walking" nor "sleeping", sets, to the scene, preset action details in accordance with a value of the action count of the scene.

11. The activity history generating system according to claim 7, wherein the activity detail analyzing module combines a first scene, a second scene, and a third scene which are successive in time series when the action details of the first scene and the action details of the third scene are the same and the second scene satisfies a given condition.

**12.** The activity history generating system according to claim 7, wherein the sensor comprises an acceleration sensor for detecting acceleration of an arm as the living organism information, and wherein the scene splitting module obtains, as the action count, a number of times the acceleration crosses a given threshold within a given time interval.

**FIG. 1**

USER

12

+Y

6

−X +X

11

−Y

(a) FRONT

12

1

10

6

10 REPRESENTS
PRINTED BOARD

+Z −Z

11

(b) SIDE

**FIG. 2**

FIG. 3

EP 2 330 554 A1

**FIG. 4**

START

S1

COLLECT SENSOR DATA

S2

SPLIT SCENES

S3

LIST ACTIVITY DETAIL CANDIDATES

S4

PRIORITIZE ACTIVITY
DETAIL CANDIDATES

S5

DISPLAY

S6

ENTER ACTIVITY DETAILS

S7

ESTABLISH ACTIVITY RECORD

S8

STORE ACTIVITY RECORD

END

**FIG. 5**

START

S11

INPUT SENSOR DATA

S12

COMPILE FOR EVERY MINUTE

S13

DETECT POINTS OF CHANGE IN ACTION

S14

SPLIT SCENES BASED ON TIME
OF POINTS OF CHANGE

END

*FIG. 6*

ZERO CROSS COUNT

ACCELERATION

↑ ACCELERATION (SCALAR)

0.05G
0G

→ TIME

NUMBER OF TIMES 0.05G IS PASSED PER UNIT TIME

*FIG. 7*

550

551    552    553    554    555    556

| USER ID | SENSOR ID | MEASUREMENT DATE/TIME | TEMPERATURE | ACTION COUNT | LEVEL OF EXERTION |
|---------|-----------|----------------------|-------------|--------------|-------------------|
|         |           |                      |             |              |                   |

*FIG. 8*

FIG. 9

START

S21

DETECT "WALKING"

S22

DETECT "SLEEPING"

S23

COMBINE WALKING SCENES

S24

COMBINE SLEEPING SCENES

S25

SET OTHER ACTION DETAILS

END

*FIG. 10*

| ACTION STATE | SLEEPING DETECTED | WALKING DETECTED | ACTION COUNT |
|---|---|---|---|
| SLEEPING | O | – | – |
| RESTING | × | × | <=50 |
| LIGHT WORK | × | × | 50>x>300 |
| WALKING | × | O | – |
| OTHER EXERCISES | × | × | >=300 |
| JOGGING | × | O | <300 |

*FIG. 11*

START

S31

FOR TWO SUCCESSIVE WALKING SCENES
W1 AND W2 WHICH DO NOT SANDWICH WALKING
SCENE, AND SCENE R1 BETWEEN W1 AND W2

S32    IS DISTRIBUTION OF
EXERTION AMOUNT EQUAL AMONG
W1, W2, AND R1 ?                         NO

YES

S33

COMBINE W1, W2, AND R1 INTO ONE WALKING SCENE

END

*FIG. 12*

START

S41

FOR TWO SUCCESSIVE SLEEPING SCENES
S1 AND S2 WHICH DO NOT SANDWICH SLEEPING SCENE

S42

IS PERIOD BETWEEN
S1 AND S2 30 MINUTES OR LESS ?

NO

YES

S43

COMBINE S1 AND S2 INTO ONE SLEEPING SCENE S

END

**FIG. 13**

FIG. 14

500

| USER ID | SCENE ID | SCENE CLASSIFICATION | START DATE/TIME | END DATE/TIME |
|---------|----------|----------------------|-----------------|---------------|
| 501 | 502 | 503 | 504 | 505 |
| 0001 | 1 | SLEEPING | 2007/01/07 01:30 | 2007/01/07 06:40 |
| 0001 | 4 | LIGHT WORK | 2007/01/07 06:40 | 2007/01/07 07:10 |
| 0001 | 6 | WALKING | 2007/01/07 07:10 | 2007/01/07 07:43 |
| 0001 | 3 | RESTING | 2007/01/07 07:43 | 2007/01/07 08:18 |
| 0001 | 6 | WALKING | 2007/01/07 08:18 | 2007/01/07 08:30 |
| 0001 | 3 | RESTING | 2007/01/07 08:30 | 2007/01/07 09:40 |
| 0001 | 6 | WALKING | 2007/01/07 09:40 | 2007/01/07 09:45 |
| 0001 | 4 | LIGHT WORK | 2007/01/07 09:45 | 2007/01/07 10:55 |
| 0001 | 6 | WALKING | 2007/01/07 10:55 | 2007/01/07 11:00 |
| 0001 | 4 | LIGHT WORK | 2007/01/07 11:00 | 2007/01/07 14:54 |
| 0001 | 6 | WALKING | 2007/01/07 14:54 | 2007/01/07 15:00 |
| 0001 | 3 | RESTING | 2007/01/07 15:00 | 2007/01/07 16:44 |
| 0001 | 6 | WALKING | 2007/01/07 16:44 | 2007/01/07 16:50 |
| ... | ... | ... | ... | ... |

*FIG. 15*

FIG. 16

600

601 602 603

| ACTIVITY CLASSIFICATION | RULE | HIT PERCENTAGE |
|---|---|---|
| COMMUTING (TO WORK) | [START DATE/TIME = 30 TO 90 MINUTES AFTER WAKING UP]<br>∧ [SCENE PATTERN = WALKING 1 – RESTING – WALKING 2]<br>∧ [WALKING 1 = 10 TO 15 MINUTES]<br>∧ [RESTING = 20 TO 25 MINUTES]<br>∧ [WALKING 2 = 7 TO 10 MINUTES] | 77% |
| LUNCH | [TIME ZONE = 12 O'CLOCK TO 13 O'CLOCK]<br>∧ [SCENE PATTERN = WALKING 1 – LIGHT WORK – WALKING 2<br>∧ [WALKING 1 = 15 TO 10 MINUTES]<br>∧ [LIGHT WORK = 20 TO 30 MINUTES]<br>∧ [WALKING 2 = 5 TO 10 MINUTES] | 82% |
| STROLLING | [START DATE/TIME = 30 TO 60 MINUTES AFTER WAKING UP]<br>[WALKING = 30 TO 60 MINUTES] | 23% |
| . . . | . . . | . . . |

**FIG. 17**

FIG. 18

**FIG. 19**

701  702  703  704

WALKING

| 9:40-11:00 INVENTORY MANAGEMENT |
| LIGHT WORK |
| WALKING |

| ACTIVITY DETAIL CANDIDATES | |
|---|---|
| INVENTORY MANAGEMENT | |
| MANUAL SELECTION | |
| WORKING | ⇒ |
| PRIVATE | ⇒ |
| SLEEPING | ⇒ |

1701

1702

1700

**FIG. 20**

1700

| ACTIVITY DETAIL CANDIDATES | |
|---|---|
| INVENTORY MANAGEMENT | |
| MANUAL SELECTION | |
| WORKING | ⇒ |
| PRIVATE | ⇒ |
| SLEEPING | ⇒ |

UPPER LEVEL

1702

| MANUAL SELECTION | |
|---|---|
| DESK WORK | ⇒ |
| TRAVELING | ⇒ |
| CONFERRING | ⇒ |

MIDDLE LEVEL

| MANUAL SELECTION | |
|---|---|
| REPARING MATERIAL | ⇒ |
| E-MAIL | |

LOWER LEVEL

**FIG. 21**

ACTIVITY DETAIL STORING TABLE

800

801 802 803 804 805 806

| ACTIVITY DETAIL ID | USER ID | START DATE/TIME | END DATE/TIME | ACTIVITY DETAIL ITEM ID | ACTIVITY DETAIL ITEM |
|---|---|---|---|---|---|
|  |  |  |  |  |  |

## FIG. 22

ACTIVITY DETAIL ITEM MANAGEMENT TABLE

900

| 901 | 902 | 903 | 904 |
|---|---|---|---|
| ACTIVITY DETAIL ITEM ID | ACTIVITY DETAIL ITEM | UPPER-LEVEL ACTIVITY DETAIL ITEM ID | UPPER-LEVEL ACTIVITY DETAIL ITEM |
| 1 | WORKING | | |
| 2 | DESK WORK | 1 | WORKING |
| 3 | PREPARING MATERIAL | 2 | DESK WORK |
| 4 | SALES PAMPHLET | 3 | PREPARING MATERIAL |
| 5 | SALES REPORT | 3 | PREPARING MATERIAL |
| 6 | E-MAIL | 2 | DESK WORK |
| . . . | . . . | | . . . |

*FIG. 23*

```
┌─────────────────────────────────────────────────┬─────┬─────┬─────┐
│ ACTIVITY DETAIL INPUT WINDOW                     │  _  │  □  │  ×  │
├─────────────────────────────────────────────────┴─────┴─────┴─────┤
│ ACTIVITY DETAILS   "INVENTORY MANAGEMENT"                          │
│ START TIME    9:40                                                 │
│ END TIME      11:00                                                │
│ COMMENT                                                            │
│     ┌───────────────────────────────────────────┐                 │
│     │ TOOK INVENTORY IN PREPARATION             │                 │
│     │  FOR END OF FISCAL YEAR                    │                 │
│     │                                           │                 │
│     │                                           │                 │
│     │                                           │                 │
│     └───────────────────────────────────────────┘                 │
│                                                                    │
│     ┌─────────────────┐         ┌─────────────────┐               │
│     │       OK        │         │     CANCEL      │               │
│     └─────────────────┘         └─────────────────┘               │
│                                                                    │
└────────────────────────────────────────────────────────────────────┘
```

700A

709

**FIG. 24**

| 801 | 802 | 803 | 804 | 805 | 806 | 807 |
|-----|-----|-----|-----|-----|-----|-----|
| ACTIVITY DETAIL ID | USER ID | START TIME/DATE | END TIME/DATE | ACTIVITY DETAIL ITEM ID | ACTIVITY DETAIL ITEM | COMMENT |
|  |  |  |  |  |  |  |

**FIG. 25**

```
┌─────────────────────────────────────────────────────┬──────┬──────┬──────┐
│ ACTIVITY DETAIL INPUT WINDOW                         │  —   │  □   │  ×   │
├─────────────────────────────────────────────────────┴──────┴──────┴──────┤
│ ACTIVITY DETAILS   "LUNCH"                                                  │
│ START TIME   9:40                                                          │
│ END TIME       11:00                                                       │
│ COMMENT                                                                    │
│  ┌──────────────────────────────────────────────────┐                     │
│  │ FRIED RICE AT ─ ─ ─ RESTAURANT                    │                     │
│  │                                                   │                     │
│  │                                                   │                     │
│  │                                                   │                     │
│  └──────────────────────────────────────────────────┘                     │
│ SCORE                                                                      │
│ TASTE           │         GOOD           │▼│                               │
│ AMOUNT ATE      │       TOO MUCH         │▼│                               │
│                                                                            │
│  ┌─────────────────┐        ┌─────────────────┐                           │
│  │      OK         │        │    CANCEL       │                           │
│  └─────────────────┘        └─────────────────┘                           │
└────────────────────────────────────────────────────────────────────────────┘
```

700A

709

710

711

**FIG. 26**

| ACTIVITY DETAIL ID | USER ID | START TIME/ DATE | END TIME/ DATE | ACTIVITY DETAIL ITEM ID | ACTIVITY DETAIL ITEM | COMMENT | SCORE 1 | SCORE 2 |
|---|---|---|---|---|---|---|---|---|
| 801 | 802 | 803 | 804 | 805 | 806 | 807 | 808 | 809 |
|  |  |  |  |  |  |  |  |  |

**FIG. 27**

ACTIVITY DETAIL INPUT WINDOW     _   □   ×

ACTIVITY DETAILS "DRINKING PARTY"
START TIME    9:40
END TIME     11:00
WITH WHOM

— 700B

| FRIENDS FROM HIGH SCHOOL |
|---|

— 712

WHERE

| A PUB BEHIND THE STATION |
|---|

— 713

WHAT

| GOT TOGETHER FOR A DRINK<br>FOR THE FIRST TIME IN A WHILE |
|---|

— 714

REMARKS

| APPRECIATED ANEW THE VALUE OF<br>OLD FRIENDSHIP |
|---|

— 715

| OK | | CANCEL |
|---|---|---|

## FIG. 28

| 801 | 802 | 803 | 804 | 805 | 806 | 810 | 811 | 812 | 813 |
|---|---|---|---|---|---|---|---|---|---|
| ACTIVITY DETAIL ID | USER ID | START TIME/ DATE | END TIME/ DATE | ACTIVITY DETAIL ITEM ID | ACTIVITY DETAIL ITEM | WITH WHOM | WHERE | WHAT | REMARKS |
| | | | | | | | | | |

## FIG. 29

1

BRACELET
TYPE
SENSOR
NODE

- - - -

102

BASE
STATION

105

104

SERVER
1500

DATA
STORING
UNIT

1600

MODULE FOR
PERFORMING
VARIOUS
ANALYSES

103

PC

1031

DISPLAY UNIT

1032

INPUT UNIT

400

DATA
STORING
UNIT

200

SCENE
SPLITTING
MODULE

300

ACTIVITY
DETAIL
ANALYZING
MODULE

**FIG. 30**

<div align="center">**INTERNATIONAL SEARCH REPORT**</div>

| | International application No. |
|---|---|
| | PCT/JP2009/064475 |

**A. CLASSIFICATION OF SUBJECT MATTER**
*G06Q50/00*(2006.01)i, *A61B5/00*(2006.01)i

According to International Patent Classification (IPC) or to both national classification and IPC

**B. FIELDS SEARCHED**

Minimum documentation searched (classification system followed by classification symbols)
G06Q50/00, A61B5/00

Documentation searched other than minimum documentation to the extent that such documents are included in the fields searched
Jitsuyo Shinan Koho         1922-1996    Jitsuyo Shinan Toroku Koho    1996-2009
Kokai Jitsuyo Shinan Koho   1971-2009    Toroku Jitsuyo Shinan Koho    1994-2009

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2005-309965 A  (Matsushita Electric Works, Ltd.), 04 November 2005 (04.11.2005), paragraphs [0030], [0053] (Family: none) | 1-12 |
| Y | Yasuhiro NIIKURA, "MPEG Fugoka Eizo Data kara no Scene Change Kenshutsu Hoho no Kento", Scene Change Detection from MPEG coded data, The Institute of Electrical Engineers of Japan Kenkyukai Shiryo, 25 September 1997 (25.09. 1997), pages 7 to 12 | 1-12 |
| Y | JP 2006-115011 A  (Sharp Corp.), 27 April 2006 (27.04.2006), paragraph [0042] (Family: none) | 1-12 |

☒  Further documents are listed in the continuation of Box C.        ☐  See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 02 November, 2009 (02.11.09) | 17 November, 2009 (17.11.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2009/064475

| | C (Continuation). DOCUMENTS CONSIDERED TO BE RELEVANT | |
|---|---|---|
| Category* | Citation of document, with indication, where appropriate, of the relevant passages | Relevant to claim No. |
| A | WO 2005/098818 A1 (Matsushita Electric Industrial Co., Ltd.), 20 October 2005 (20.10.2005), entire text; all drawings & US 2007/0154032 A1 & WO 2005/098818 A1 | 1-12 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006209468 A
- JP 2005346291 A
- JP 2005062963 A
- JP 2004287539 A
- JP 2008000283 A
- JP 2008059058 A

**Non-patent literature cited in the description**

- **Cole RJ ; Kripke DF ; Gruen W ; Mullaney DJ ; Gillin JC.** Automatic Sleep/Wake Identification from Wrist Activity. *Sleep,* 1992, vol. 15, 491-469